(19) Europäisches Patentamt
European Patent Office
Office européen des brevets

(11) Publication number: 0 189 774 B1

# (12) EUROPEAN PATENT SPECIFICATION

(45) Date of publication of the patent specification: **12.04.89**

(51) Int. Cl.⁴: **C 07 B 43/06**, C 07 C 102/00, C 07 D 227/00, C 07 D 247/00, C 07 D 269/00, C 07 D 283/00, C 07 D 325/00, C 07 D 333/00

(21) Application number: **86100362.2**

(22) Date of filing: **13.01.86**

## (54) Process for production of N,N-disubstituted carboxylic acid amides.

(30) Priority: **16.01.85 JP 4107/85**

(43) Date of publication of application: **06.08.86 Bulletin 86/32**

(45) Publication of the grant of the patent: **12.04.89 Bulletin 89/15**

(84) Designated Contracting States: **DE FR GB**

(56) References cited:
**JOURNAL OF ORGANIC CHEMISTRY, vol. 27, July 1962, pages 2640-2643; J.H. BILLMAN et al.: "The reductive acylation of Schiff bases using trimethylamine borane. IV"**

(73) Proprietor: **TOKUYAMA SODA KABUSHIKI KAISHA, 1-1 Mikage-cho, Tokuyama-shi Yamaguchi-ken (JP)**

(72) Inventor: **Kato, Shozo, 1090, Endo Fujisawa-shi, Kanagawa-ken (JP)**
Inventor: **Okamoto, Hidenori, 7-52-1, Shonandai, Fujisawa-shi Kanagawa-ken (JP)**

(74) Representative: **Kraus, Walter, Dr. et al, Patentanwälte Kraus, Weisert & Partner Thomas-Wimmer-Ring 15, D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to a novel process for producing N,N-disubstituted carboxylic acid amides of the following formula (I)

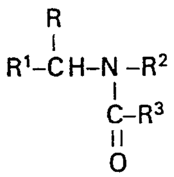

(I)

wherein R, $R^1$, $R^2$ and $R^3$ are defined below.

Processes for producing N,N-disubstituted carboxylic acid amides of formula (I) which are useful, for example, as medicines, agricultural chemicals, or starting materials or intermediates for producing them are generally known [see, for example, Japanese Laid-Open Patent Publication No. 4181/1985 laid-open on January 10, 1985; U.S. Patents Nos. 2 863 752, 4 372 972, 4 282 028, 4 460 603, 4 521 243, 4 494 978, 4 155 733, 4 456 471, 3 586 496 and 3 367 847; R.B. Wagner, H.D. Zook, «Synthetic Organic Chemistry», p. 565 (1953), John Wiley & Sons, Inc.; and A. Venkov, M. Nikolova, N. Mollov, Chem. Ind. (London), p. 808 (1982)].

The most general industrial process among them comprises reducing a Schiff base of the following formula (II)

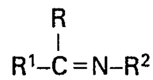

(II)

wherein R, $R^1$ and $R^2$ are as defined below, and acylating or aroylating the resulting reduction product to form a compound of formula (I). For example, the above-cited Japanese Laid-Open Patent Publication No. 4181/1985 discloses a process for producing N,N-disubstituted carboxylic acid amides by a step of reducing the starting Schiff base and a step of chloroacetylating the resulting reduction product in accordance with the following scheme.

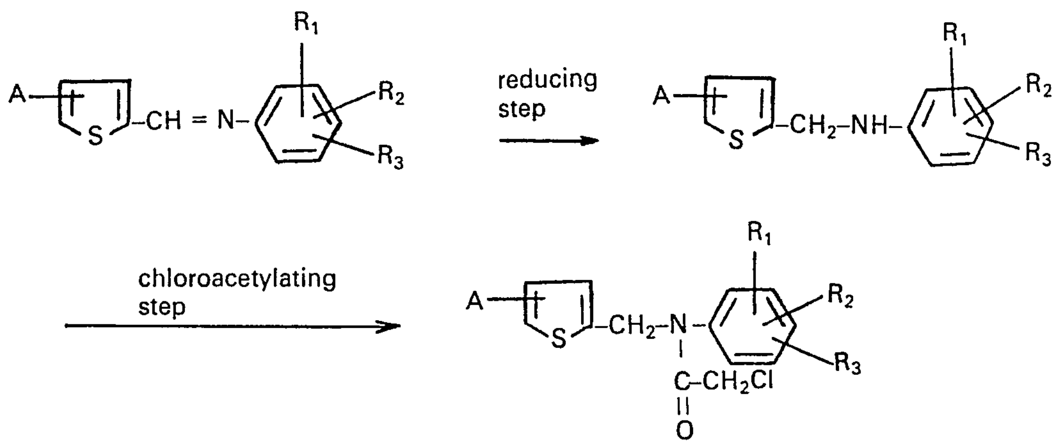


In the above scheme, A represents a hydrogen atom, a halogen atom, an alkyl group, an alkoxy group or an alkylthio group, and $R_1$, $R_2$ and $R_3$, independently from each other, represent a hydrogen atom, a halogen atom, an alkyl group, an alkoxy group or an alkylthio group.

Known processes for producing N,N-disubstituted carboxylic acid amides including the above-exemplified process have one or more defects. For example, they have complex manufacturing steps. The compounds used as starting materials or intermediates are difficult to obtain, or are unstable. Furthermore, the yields of the desired compounds are low. These defects make it difficult for the known processes to give the desired compounds in good yields at low cost by simple manufacturing steps.

The present inventors have extensively worked on an improvement in conventional industrial processes for producing N,N-disubstituted carboxylic acid amides which essentially comprise the above two steps, and consequently found that by contacting a Schiff base with a carboxylic acid derivative in the presence of a silane compound, the desired N,N-disubstituted carboxylic acid amide can be produced easily in good yields by a one-step process. The investigations of the present inventors have shown that this novel mode of reaction can be applied to the reaction of a wide range of Schiff bases with a wide range of carboxylic acid derivatives.

According to this invention, there is provided a novel process for producing N,N-disubstituted carboxylic acid amides represented by the following formula (I)

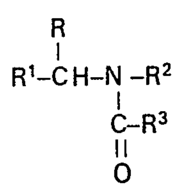

(I)

wherein R represents a hydrogen atom, an alkyl group, an alkenyl group, an alkoxy group, an alkenyloxy group, alkylcarbonyl groups having alkyl with 1 to 8 carbon atoms, alkoxycarbonyl groups having alkoxy with 1 to 8 carbon atoms, and arylcarbonyl groups having substituted or unsubstituted aryl having 6 to 10 carbon atoms, or a triha-

logenomethyl group; $R^1$, $R^2$ and $R^3$, independently from each other, represent a substituted or unsubstituted alkyl group, a substituted or unsubstituted alkenyl group, a substituted or unsubstituted alkynyl group, a substituted or unsubstituted aryl group, a substituted or unsubstituted heteroaryl group, a substituted or unsubstituted cycloalkyl group, a substituted or unsubstituted cycloalkenyl group or a substituted or unsubstituted heterocycloalkyl group; $R^1$ may further represent a hydrogen atom; and R and $R^1$, taken together, may form a substituted or unsubstituted cyclic group together with the carbon atom to which they are bonded, which comprises contacting a Schiff base represented by the following formula (II)

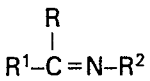
(II)

wherein R, $R^1$ and $R^2$ are as defined above, with a carboxylic acid derivative represented by the following formula (III)

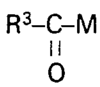
(III)

wherein $R^3$ is as defined above, and M represents a halogen atom or the moiety 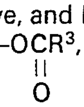, in the presence of a silane compound represented by the following formula (IV)

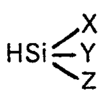
(IV)

wherein X, Y and Z, independently from each other, represent a hydrogen atom or a halogen atom, providing that at least one of X, Y and Z is halogen, in the presence or absence of an inert organic solvent.

The novel process of this invention can be shown, for example, by the following reaction scheme.

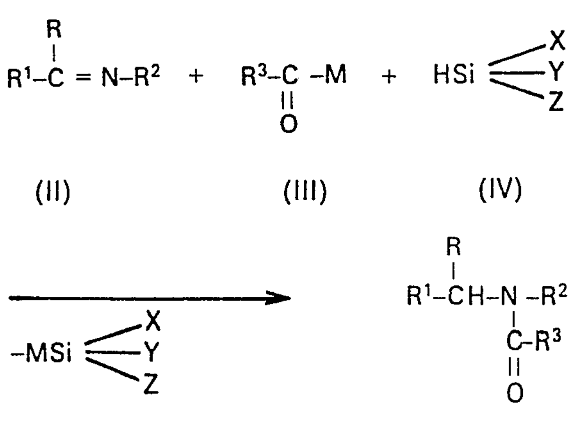
(II) (III) (IV)

(I)

The mechanism of the reaction by which the desired compound of formula (I) is formed in one step by the above novel process is not entirely clear. One possible mechanism presumed by the present inventors is that the Schiff base (II) and the carboxylic acid derivative (III) react in the presence of the silane compound (IV) to form an immonium salt-type compound

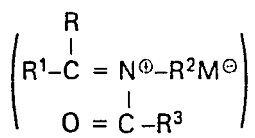

or its M adduct

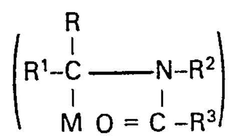

as an intermediate which further reacts with the silane compound (IV) to form the desired amide compound. Another possible mechanism is that the Schiff base (II) first reacts with the silane compound (IV) to form an N-silyl compound

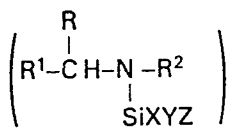

after which its acylation or aroxylation takes place to form the desired amide compound.

It should be understood that the above reaction mechanisms are merely presumed, and do not in any way limit the novel process of this invention, and that the process of this invention includes all embodiments in which the desired compound of formula (I) is formed in one step by «contacting of the compound of formula (II) with the compound of formula (III) in the presence of the compound (IV)» which results in the reaction of these three components.

It has thus been found in accordance with this invention that by the novel mode of reaction, the compound of formula (I) can be produced easily in good yields at low cost with industrial advantage from the compound of formula (II) in one step.

It is an object of this invention to provide a novel process for producing N,N-disubstituted carboxylic acid amides of formula (I).

The above and other objects and advantages of this invention will become apparent from the following description.

According to the process of this invention, the N,N-disubstituted carboxylic acid amides of formula (I) can be produced by contacting the Schiff base of formula (II) with the carboxylic acid derivative of formula (III) in the presence of the silane compound of formula (IV) in the presence or absence of an inert organic solvent. In formulae (I)

and (II), R represents a hydrogen atom, an alkyl group, an alkenyl group, an alkoxy group, an alkenyloxy group, a substituted carbonyl group or a trihalogenomethyl group.

The alkyl group may, for example, be an alkyl group having 1 to 10 carbon atoms, preferably 1 to 6 carbon atoms. Specific examples include methyl, ethyl, propyl, butyl, pentyl, hexyl and octyl groups. The alkenyl group may, for example, be an alkenyl group having 2 to 6 carbon atoms, preferably 2 to 4 carbon atoms. Specific examples of the alkenyl group are vinyl, allyl, propenyl, butenyl, pentenyl and hexenyl groups. The alkoxy group may, for example, be an alkoxy group having 1 to 6 carbon atoms, preferably 1 to 4 carbon atoms. Specific examples of the alkoxy group are methoxy, ethoxy, propoxy, butoxy, pentoxy, hexoxy and octoxy groups. The alkenyloxy group may, for example, be an alkenyloxy group having 2 to 6 carbon atoms, preferably 2 to 4 carbon atoms. Specific examples of the alkenyloxy group are vinyloxy, allyloxy, propenyloxy, butenyloxy, pentenyloxy and hexenyloxy groups. The alkylcarbonyl groups have preferably 1 to 4 carbon atoms, and the alkoxycarbonyl groups have preferably 1 to 4 carbon atoms. In the above arylcarbonyl groups, examples of the substituent for the substituted aryl include alkyl groups having 1 to 6 carbon atoms, alkenyl groups having 2 to 6 carbon atoms, alkynyl groups having 2 to 6 carbon atoms, alkoxy groups having 1 to 6 carbon atoms, alkylthio groups having 1 to 6 carbon atoms, alkoxycarbonyl groups having 1 to 4 carbon atoms in the alkoxy moiety, alkylcarbonyl groups having 1 to 4 carbon atoms in the alkyl moiety, halogen atoms, a cyano group, a nitro group, dialkylamino groups having 1 to 4 carbon atoms in each alkyl group, trihalogenomethyl groups and alkylcarbonyloxy groups having 1 to 4 carbon atoms in the alkyl moiety. Examples of the aryl group having 6 to 10 carbon atoms are phenyl, naphthyl, tetrahydronaphthyl and indanyl groups.

Thus, specific examples of such substituted carbonyl groups include methylcarbonyl, ethylcarbonyl, propylcarbonyl, butylcarbonyl, methoxycarbonyl, ethoxycarbonyl, propoxycarbonyl, butoxycarbonyl, phenylcarbonyl, naphthylcarbonyl, chlorophenylcarbonyl, bromophenylcarbonyl, fluorophenylcarbonyl, dichlorophenylcarbonyl, chlorofluorophenylcarbonyl, methoxyphenylcarbonyl, ethoxyphenylcarbonyl, methylthiophenylcarbonyl, cyanophenylcarbonyl, nitrophenylcarbonyl, (methylcarbonyloxy)phenylcarbonyl, (trifluoromethyl)phenylcarbonyl, (diethylamino) phenylcarbonyl, (ethoxycarbonyl)phenylcarbonyl, chloronaphthylcarbonyl, and ethoxynaphthylcarbonyl groups. Examples of the trihalogenomethyl group for R are trihalogenomethyl groups in which the halogen is selected from Cl, Br, I and F. Specific examples of such trihalogenomethyl groups are trichloromethyl, tribromomethyl and trifluoromethyl groups.

In formulae (I), (II) and (III), $R^1$, $R^2$ and $R^3$, independently from each other, represent a substituted or unsubstituted alkyl groups, a substituted or unsubstituted alkenyl group, a substituted or unsubstituted alkynyl group, a substituted or unsubstituted aryl group, a substituted or unsubstituted heteroaryl group, a substituted or unsubstituted cycloalkyl group, a substituted or unsubstituted cycloalkenyl group or a substituted or unsubstituted heterocycloalkyl group; $R^1$ may further represent a hydrogen atom; and R and $R^1$, taken together, may form a substituted or unsubstituted cyclic group together with the carbon atom to which they are bonded.

Preferred examples of $R^1$, $R^2$ and $R^3$ include substituted or unsubstituted alkyl groups having 1 to 15 carbon atoms, preferably 1 to 10 carbon atoms, substituted or unsubstituted alkenyl groups having 2 to 15 carbon atoms, preferably 2 to 10 carbon atoms, substituted or unsubstituted alkynyl groups having 2 to 15 carbon atoms, preferably 2 to 10 carbon atoms, substituted or unsubstituted aryl groups having 6 to 20 carbon atoms, preferably 6 to 15 carbon atoms, substituted or unsubstituted heteroaryl groups having 2 to 20 carbon atom preferably 2 to 14 carbon atoms, and containing 1 to 4 hetero atoms selected from nitrogen, oxygen and sulfur atoms, substituted or unsubstituted cycloalkyl groups having 3 to 20, preferably 3 to 15 carbon atoms, substituted or unsubstituted cycloalkenyl groups having 3 to 20 carbon atoms, preferably 3 to 15 carbon atoms, and substituted or unsubstituted heterocycloalkyl groups having 2 to 20 carbon atoms, preferably 2 to 15 carbon atoms, and containing 1 to 4 hetero atoms selected from nitrogen, oxygen and sulfur atoms.

Examples of the substituted or unsubstituted cyclic group formed by R and $R^1$ together with the carbon atom to which they are bonded include cycloalkyl groups having 4 to 15 carbon atoms, preferably 5 to 10 carbon atoms, and heterocycloalkyl groups having 4 to 15 carbon atoms preferably 5 to 10 carbon atoms, and containing 1 to 4 nitrogen, oxygen or sulfur atoms. Specific examples of such cyclic groups are cyclopentyl, cyclohexyl, cycloheptyl, pyrrolidyl, piperidyl, tetrahydrofuryl and tetrahydropyryl groups.

In the illustrated groups for $R^1$, $R^2$, and $R^3$, examples of substituents which they may have include alkyl groups having 1 to 6 carbon atoms, alkenyl groups having 2 to 6 carbon atoms, alkynyl groups having 2 to 6 carbon atoms, alkoxy groups having 1 to 6 carbon atoms, alkylthio groups having 1 to 6 carbon atoms, alkoxycarbonyl groups having 1 to 4 carbon atoms, alkylcarbonyl groups having 1 to 4 carbon atoms, halogen atoms, a cyano group, a nitro group, dialkylamino groups having 1 to 4 carbon atoms in each alkyl moiety, trihalogenomethyl groups and alkylcarbonyloxy groups having 1 to 4 carbon atoms in the alkyl moiety.

More specific examples of the organic groups $R^1$, $R^2$ and $R^3$ which are widely used industrially are given below.

The unsubstituted alkyl groups include linear or branched alkyl groups, such as methyl, ethyl, propyl, butyl, pentyl, hexyl, heptyl, octyl, nonyl, decyl, undecyl and dodecyl.

The substituted alkyl groups include linear or branched haloalkyl groups such as fluoromethyl, trifluoromethyl, chloromethyl, trichloromethyl, chloroethyl, bromoethyl, fluoropropyl, chloropropyl, chlorobutyl, bromopentyl, chlorohexyl, fluorooctyl, trifluoroethyl and heptafluorobutyl; linear or branched alkoxyalkyl groups such as methoxymethyl, methoxyethyl, methoxypropyl, methoxybutyl, methoxypentyl, methoxyhexyl, ethoxymethyl, ethoxyethyl,ethoxypropyl, ethoxybutyl, propoxymethyl, propoxyethyl, propoxypropyl, propoxybutyl, butoxymethyl, butoxyethyl, butoxypropyl, butoxybutyl and pentoxyethyl; phenoxyalkyl groups such as phenoxymethyl, phenoxyethyl, chlorophenoxypropyl and trichlorophenoxypropyl; cyanoalkyl groups such as cyanoethyl, cyanopropyl and cyanobutyl; nitroalkyl groups such as nitroethyl, nitropropyl, nitrohexyl and nitrodecyl; alkylthioalkyl groups such as methylthiomethyl, methylthioethyl, methylthiopropyl, ethylthiomethyl, ethylthioethyl, ethylthiobutyl and propylthioethyl; arylalkyl groups such as phenylmethyl, phenylethyl, phenylpropyl, methylphenylmethyl and chlorophenylmethyl; heteroarylalkyl groups such as thienylmethyl, thienylethyl, methoxythienylmethyl, furylmethyl, furylethyl, chlorofurylmethyl, pyrrolylmethyl pyrazolylmethyl and pyrazolylethyl; cycloalkylalkyl groups such as cyclopropylmethyl and cyclohexylethyl; and alkoxycarbonylalkyl groups such as methoxycarbonylmethyl, methoxycarbonylethyl, ethoxycarbonylmethyl, ethoxycarbonylethyl and ethoxycarbonylpropyl.

The unbstituted alkenyl groups include various position isomers of ethenyl, propenyl, butenyl, pentenyl, hexenyl and octenyl.

The substituted alkenyl groups include haloalkenyl groups such as chloroethenyl, fluoroethenyl, bromoporopenyl, chlorobutenyl, chloropentenyl and fluorohexenyl; alkoxyalkenyl groups such as methoxyethenyl, methoxypropenyl, ethoxybutenyl, ethoxyhexenyl and propoxybutenyl; and other substituted alkenyl groups including cyanoethenyl, cyanopropenyl, nitropropenyl, dimethylaminoethenyl, furylethenyl and methylthiopropenyl.

The unsubstituted alkynyl groups include ethynyl, propynyl, butynyl, pentynyl and hexynyl.

The substituted alkynyl groups include chloropropynyl, bromobutynyl, methoxybutynyl, cyanopropynyl and methylthiobutynyl.

The unsubstituted aryl groups include phenyl, naphthyl anthranyl and phenanthrenyl.

The substituted aryl groups include alkylphenyl groups such as methylphenyl, dimethylphenyl, ethylphenyl, diethylphenyl, propylphenyl, dipropylphenyl, butylphenyl, pentylphenyl, hexylphenyl, methyl(ethyl)phenyl, methyl(propyl)phenyl, ethyl(propyl)phenyl, methylbutylphenyl, di(bromoethyl)phenyl and (trifluoromethyl)phenyl; halophenyl groups such as fluorophenyl, difluorophenyl, chlorophenyl, dichlorophenyl, bromophenyl, iodophenyl, trichlorophenyl and chlorofluorophenyl; alkoxyphenyl groups such as methoxyphenyl, dimethoxyphenyl, trimethoxyphenyl, ethoxyphenyl, diethoxyphenyl, propoxyphenyl and butoxyphenyl; substituted phenyl groups such as cyanophenyl, nitrophenyl, chloro(methyl)phenyl, chloro(ethoxy)phenyl, methyl-(methoxy)phenyl, methylthiophenyl, (trifluoromethyl)phenyl, bis(chloroethylamino)phenyl, nitro(methyl)phenyl, diphenyl, chloro(dimethyl)-phenyl, (dimethylamino)phenyl, ethynylphenyl, chloro(methoxy)phenyl, methyl(propoxy)phenyl, (chloroacetyl)phenyl, methyl(butoxy)phenyl, methylcarbonyloxyphenyl and acetylphenyl; and substituted naphthyl groups such as methylnaphthyl, dimethylnaphthyl, ethylnaphthyl, chloronaphthyl, dichloronaphthyl, methoxynaphthyl, methylthionaphthyl, nitronaphthyl and cyanonaphthyl.

The unsubstituted heteroaryl groups include furyl, thienyl, pyrrolyl, pyridyl, pyrimidyl, benzofuryl, benzothienyl, indolyl, quinolyl, thiazolyl, pyrazolyl, benzothiazolyl, thiadiazolyl and oxazolyl.

The substituted heteroaryl groups include substituted furyl groups such as methylfuryl, dimethylfuryl, ethylfuryl, propylfuryl, chlorofuryl, bromofuryl, methoxyfuryl, ethoxyfuryl, propoxyfuryl, methylthiofuryl, ethylthiofuryl and nitrofuryl; substituted thienyl groups such as methylthienyl, ethylthienyl, propylthienyl, butylthienyl, fluorothienyl, chlorothienyl, bromothienyl, iodothienyl, methoxyethienyl, ethoxythienyl, propoxythienyl, methylthiothienyl, ethylthiothienyl, nitrothienyl, dichlorothienyl and dimethylthienyl; substituted pyrrolyl groups such as N-methylpyrrolyl, N-ethylpyrrolyl, methyl-N-methylpyrrolyl, chloro-N-ethylpyrrolyl, methoxy-N-methylpyrrolyl, methoxypyrrolyl, ethylpyrrolyl and chloropyrrolyl; substituted pyridyl groups methylpyridyl, ethylpyridyl, chloropyridyl and methoxypyridyl; substituted benzofuryl groups such as methylbenzofuryl, chlorobenzofuryl, ethoxybenzofuryl, nitrobenzofuryl, bromo(methoxy)benzofuryl and chloromethylbenzofuryl; substituted benzothienyl groups such as ethylbenzoethienyl, fluorobenzothienyl, methoxybenzothienyl, nitrobenzothienyl and chlorobenzothienyl; substituted quinolyl groups such as methylquinolyl, ethylquinolyl, chloroquinolyl and methoxyquinolyl; and other substituted heteroaryl groups including methylthiazolyl, methylindolyl, methylpyrimidyl, methylisothiazolyl, ethylcarbazolyl, dimethylpyrrolyl, methylisoxazolyl, phenylisoxazolyl, methoxydihydropyrimidyl, methylthiazolyl, methyloxadiazolyl, N-methyl(methylthio)triazolyl, methylthiothiadiazolyl, methylthiazolyl, and methylthiazinyl.

The unsubstituted cycloalkyl groups include cyclopropyl, cyclobutyl, cyclopentyl and cyclohexyl.

The substituted cycloalkyl groups include methylcyclopropyl, ethylcyclopropyl, propylcyclopropyl, chlorocyclopropyl, methoxycyclopropyl, ethoxycyclopropyl, methylcyclobutyl, bromocyclobutyl, methylthiocyclobutyl, chlorocyclopentyl, methylcyclohexyl, ethylcyclohexyl, chlorocyclohexyl, methoxycyclohexyl, propoxycyclohexyl,

dimethylcyclohexyl, dichlorocyclopropyl, chlorocyclohexyl, tetrahydronaphthyl and dihydroindanyl.

The unsubstituted cycloalkenyl groups include cyclobutenyl, cyclopentenyl and cyclohexenyl.

The substituted cycloalkenyl groups include methylcyclobutenyl, chlorocyclopentenyl, methoxycyclopentenyl, methylcyclohexenyl, ethylcyclohexenyl, chlorocyclohexenyl, methoxycyclohexenyl, ethoxycyclohexenyl, trimethylcyclohexenyl, dimethylcyclohexenyl, tetramethylcyclohexenyl and propenylcyclohexenyl. The unsubstituted heterocycloalkyl groups include tetrahydrofuryl, tetrahyrothienyl, pyrrolidyl, tetrahydropyryl, tetrahydrothiopyryl and piperidyl.

The substituted heterocycloalkyl groups include N-methylpyrrolidyl, N-ethylpyrrolidyl, N-methylpiperidyl, dihydropyryl, dimethylpiperidyl, dioxolanyl and N-ethylpiperidyl.

Compounds having the above-listed groups, in many cases, have various position isomers which may equally be used in the practice of the process of this invention without any particular limitation. For example, the methylphenyl groups includes o-methylphenyl, m-methylphenyl and p-methylphenyl groups, and the butyl group includes n-butyl, sec-butyl and tert-butyl groups.

The substituents are not limited to the above specific examples. Any substituents may be used as required so long as they lead to the formation of the carboxylic acid amides of formula (I) contemplated by the process of this invention.

The Schiff bases of formula (II) which are preferred in industrial practice may be grouped into the following compounds.

Compounds of the following formula

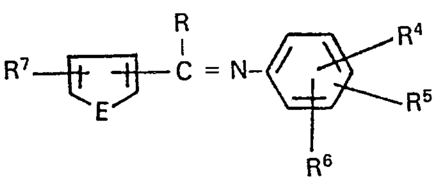

(II-1)

wherein E represents O or S, R is as defined above, $R^4$, $R^5$, $R^6$ and $R^7$, independently from each other, are the same as defined above for $R^1$, provided that they do not form a cyclic group with R.

Compounds of the following formula

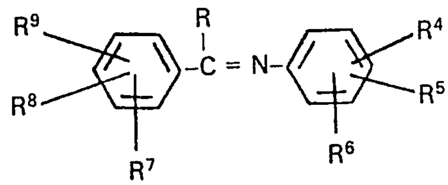

(II-2)

wherein R is as defined above, and $R^4$, $R^5$, $R^6$, $R^7$, $R^8$ and $R^9$, independently from each other, are the same as defined above for $R^1$, provided that they do not form a cyclic group with R.

Compounds of the following formula

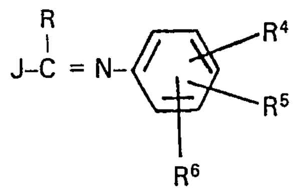

(II-3)

wherein J represents an alkyl, alkoxy alkoxyalkyl or alkoxycarbonyl group, R is the same as defined above, and $R^4$, $R^5$ and $R^6$, independently from each other, are the same as defined above for $R^1$.

The following compounds may be cited as specific examples of the Schiff base (II) which can be advantageously used in industrial practice. In the following compounds, Et represents an ethyl group.

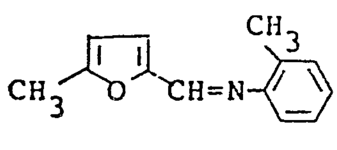 , 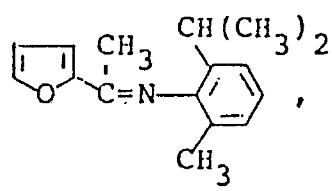 , 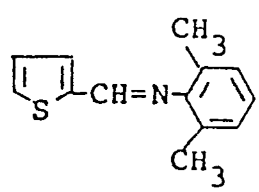 ,

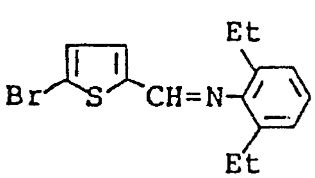 , 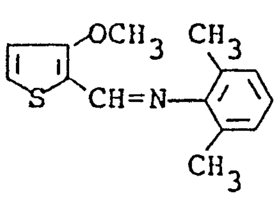 , 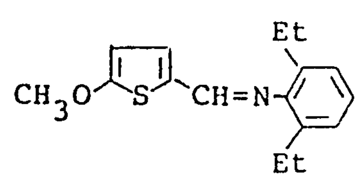 ,

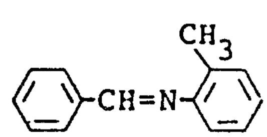 , 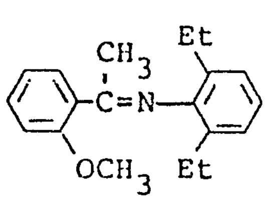 , 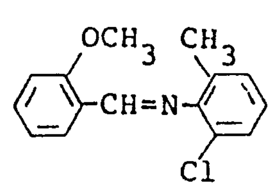 ,

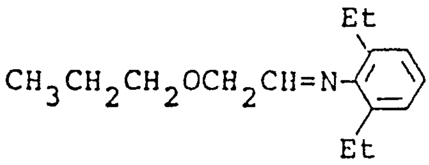 , 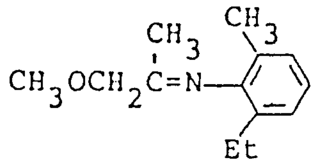 , 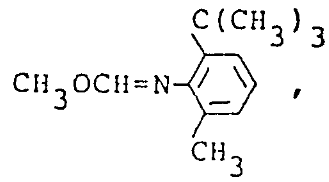 ,

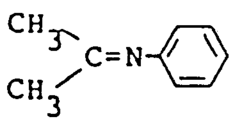 , 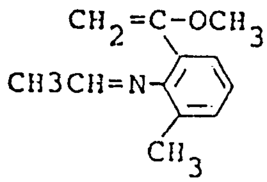 , 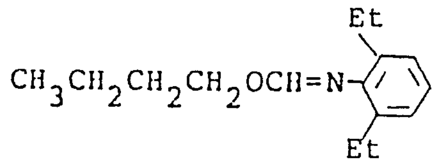 ,

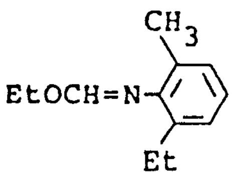 , 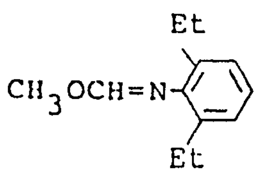 , 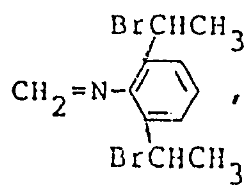 ,

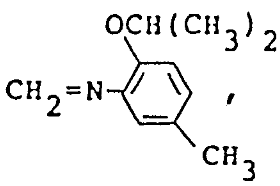 , 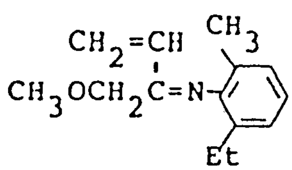 , 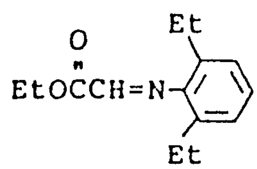 ,

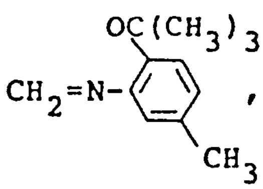 , 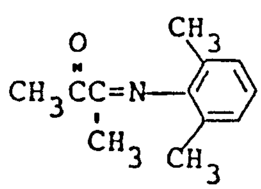 , 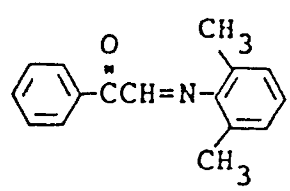 ,

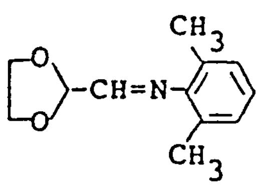 , 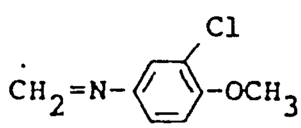 , 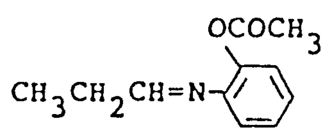 ,

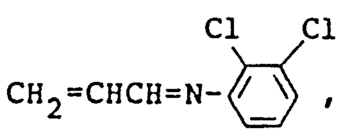 , 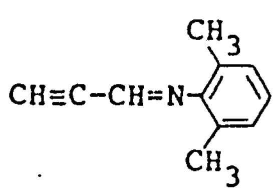 , 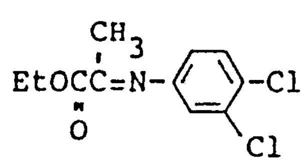 ,

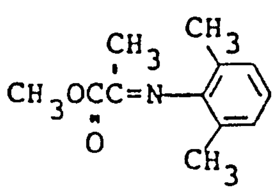 , 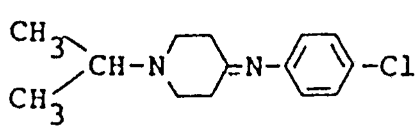 , 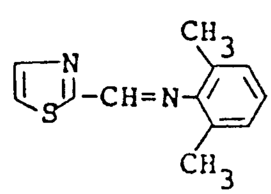 ,

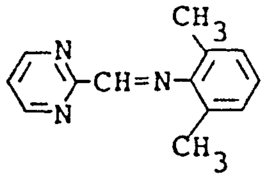 , 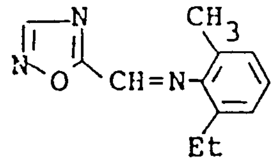 , 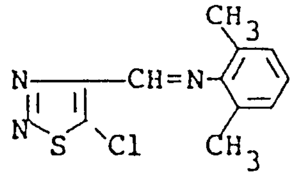 ,

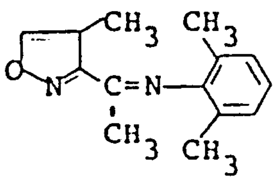 , 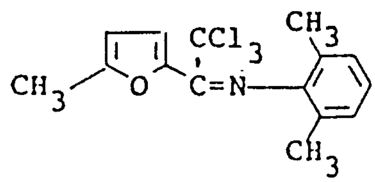 , 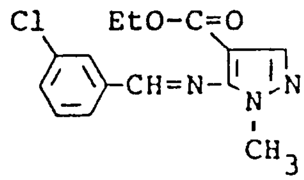 ,

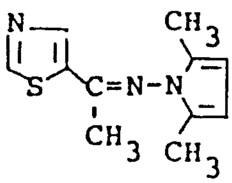 , 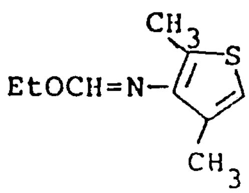 , 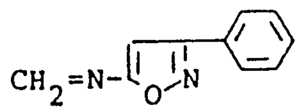 ,

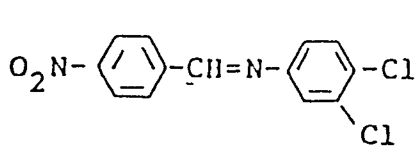 , 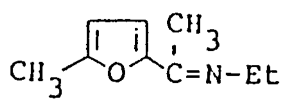 ,

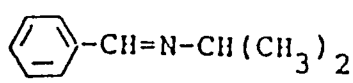 , 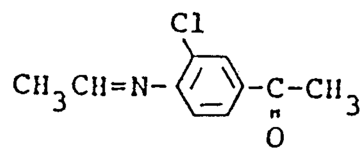 ,

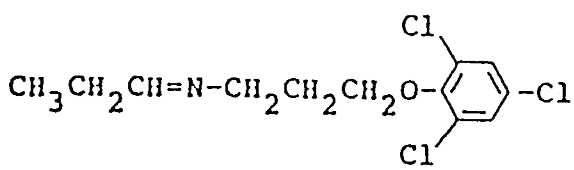 , 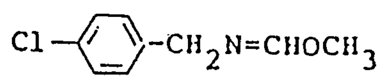 ,

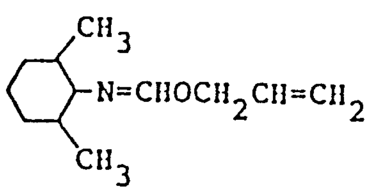 , 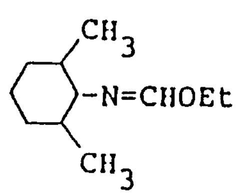 ,

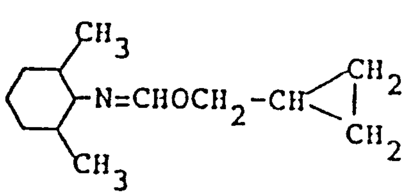 , 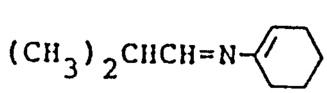 ,

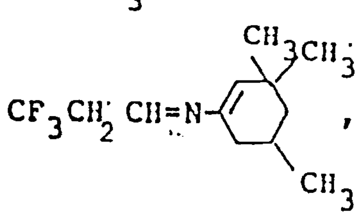 , 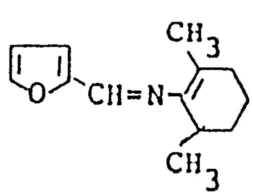 , 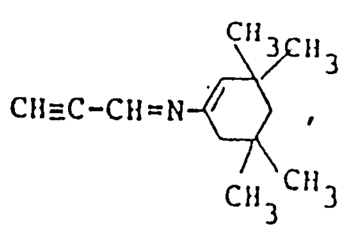 ,

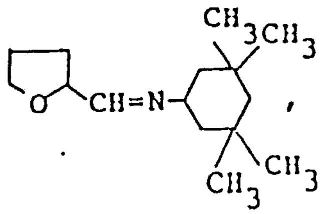, 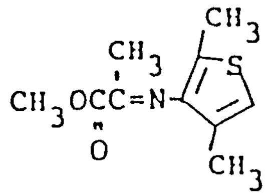, 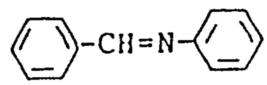, 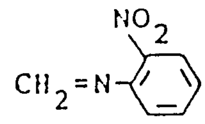,

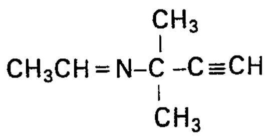, $CH_3CH_2CH_2CH=N-Et$, 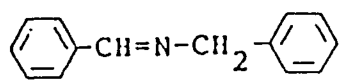,

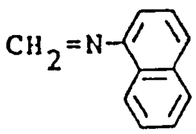, 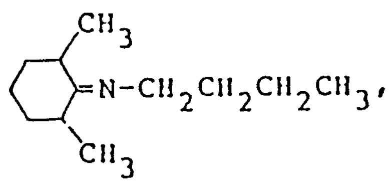, 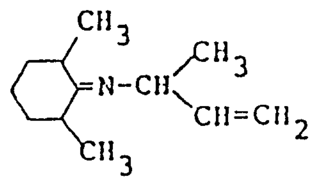

In the silane compound of the following formula (IV)

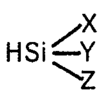 (IV)

wherein X, Y and Z, independently from each other, represent a hydrogen atom or a halogen atom providing that at least one of X, Y and Z is halogen, the halogen atom is chlorine, bromine, iodine or fluorine. Chlorine and bromine are preferred. Specific examples of the silane compounds which are preferably used in industrial practice are $HSiCl_3$, $HSiBr_3$, $H_2SiCl_2$, $H_2SiBr_2$ and $H_3SiBr$.

The carboxylic acid derivative of the following formula

$R^3$–C–M
||
O (III)

wherein $R^3$ is as defined above and M represents a halogen atom or the moiety –OCR$^3$, is a carboxylic acid halide when M in formula (III) is a halogen atom. Examples of the halogen atom are fluorine, chlorine, bromine and iodine atoms. For industrial practice, carboxylic acid chlorides or bromides of the above formula in which M is a chlorine or bromine atom are especially preferred. When M represents the moiety –OCR$^3$, the above formula (III) represents a carboxylic acid anhydride

$R^3$–C–O–C–$R^3$.
|| ||
O O

Examples of preferred compounds (III) are compounds of the following formulae (III-1) and (III-2).

Compounds of the formula

$GCH_2$–C–M
||
O (III-1)

wherein M is as defined above, and G represents a hydrogen atom, a halogen atom, an alkyl group or an alkoxy group.

Compounds of the formula

L–C–M
||
O (III-2)

wherein M is as defined above, and L represents a phenyl, pyridyl, furyl or benzyl group.

Examples of the halogen atom for G in formula (III-1) are chlorine, bromine, iodine and fluorine. Examples of the alkyl groups for G are those having 1 to 10 carbon atoms, and examples of the alkoxy group for G are those having 1 to 10 carbon atoms.

Specific examples of the compound of formula (III) include carboxylic acid halides such as acetyl chloride, acetyl bromide, chloroacetyl chloride, dichloroacetyl chloride, bromoacetyl bromide, iodoacetyl chloride, dibromoacetyl chloride, methoxyacetyl chloride, cyanoacetyl chloride, trifluoroacetyl fluoride, propionyl chloride, chloropropionyl chloride, bromopropionyl bromide, butanoyl chloride, chlorbutanoyl chloride, pentanoyl chloride, chloropentanoyl chloride, hexanoyl chloride, acryloyl chloride, butenoyl chloride, chlorobutenoyl chloride, pentenoyl chloride, chloropentenoyl chloride, propyoyl chloride, phenylpropionyl chloride, phenylpropenoyl chloride, furylacryloyl chloride, benzoyl chloride, benzoyl bromide, methylbenzoyl chloride, ethylbenzoyl bromide, chlorobenzoyl chloride, fluorobenzoyl fluoride, methoxybenzoyl chloride, chloro(methyl)benzoyl chloride, cyanobenzoyl chloride, phenylbenzoyl chloride, nitrobenzoyl chloride, furoyl chloride, thiophenecarboxylic acid chloride, indolecarboxylic acid chloride, picolyl chloride, cyclopropanecarboxylic acid chloride, phenylacetyl chloride, thienylacetyl chloride, dimethylbenzoyl chloride, fluoroacetyl chloride, dibromoacetyl bromide, phenylchloroacetyl chloride, phenyldichloroacetyl chloride, ethoxybutanoyl chloride, cyanopentanoyl chloride, nitropropionyl bromide, methylthiophenecarboxylic acid chloride, pyrazolylacetyl chloride, pyridinecarboxylic acid chloride, methylthiopyridinecarboxylic acid chloride, acetoacetyl bromide, methylthiopropionyl bromide, bromofuranecarboxylic acid bromide, piperidinecarboxylic acid chloride, ethoxycarbonylacetyl chloride, heptafluoropropoxytetrafluoropropionyl fluoride, imidazolecarboxylic acid chloride, and cinnolinecarboxylic acid chloride; and carboxylic acid anhydrides such as acetic anhydride, propionic anhydride, trifluoroacetic anhydride, chloroacetic anhydride and benzoic anhydride.

In the practice of the process of this invention the reaction may be carried out in the presence or absence of a solvent. Generally, it is preferred to carry it out in an inert organic solvent which does not react with the reactants and the products. Examples of the inert organic solvent include hydrocarbons, halogenated hydrocarbons, and nitriles, such as benzene, toluene, methylene dichloride, chloroform and acetonitrile.

The amounts of the compounds of formulae (II), (III) and (IV) charged in the practice of the process of this invention can be properly selected. For example, the silane compound of formula (IV) is used in an amount of 0.34 to 2.2 moles per mole of the Schiff base of formula (II). This amount may be varied properly depending upon the number of hydrogen atoms which the silane compound of formula (IV) has. For example, when the silane compound of formula (IV) has one hydrogen atom, its amount is 1 to 2.2 moles. Likewise, its amount is 0.5 to 1.7 moles for 2 hydrogen atoms, and 0.34 to 1.5 moles for 3 hydrogen atoms. The amount of the compound of formula (III) is, for example, 1 to 1.2 moles per mole of the Schiff base of formula (II). The amounts of the compounds of formulae (II), (III), and (IV) can be easily set at preferred values by preliminary experiments according to the types of these compounds, the reaction conditions, etc.

If the carboxylic acid derivative of formula (III) is used in too large an amount, side-reactions sometimes tend to occur. Accordingly, it is preferred to determine the mole ratio of the starting materials experimentally before they are actually used in the process.

The Schiff base of formula (II), as one starting material used in the invention, needs not to be a purified one. For example, it is possible to synthesize a Schiff base from an aldehyde and an amine by azeotropic dehydration, etc., and the as-synthesized Schiff base can be reacted directly with the silane compound (IV) and the carboxylic acid derivative (III).

The reaction temperature is not particularly limited, and can be selected from a broad range of temperatures. Preferably, the reaction is carried out at temperatures which are suitable in view of the chemical reactivity of the starting cpounds or the stability of the resulting amide compound. Generally, it is selected from a range of −20 °C to 150 °C. The reaction time, which varies depending upon the reaction temperature, may generally be several minutes to several days, for example 5 minutes to 10 days.

The sequence of addition of the reactants of formulae (II), (III) and (IV) in the process of this invention is not particularly limited. Generally, the silane compound and the carboxylic acid derivative are added to the Schiff base at room temperature or under cooling. It is also possible to add the Schiff base to a solution of the silane compound and the carboxylic acid derivative. In general, a solvent is frequently used in this reaction. For example, it is possible to add the Schiff base, the silane compound and the carboxylic acid derivative to a solvent, and then react the three compounds in it. Alternatively, the three compounds are dissolved in separate solvents and the solutions are mixed for reaction.

According to this invention, the carboxylic acid amides of formula (I) can be easily obtained by reacting the starting compounds of formulae (II), (III) and (IV). There is no particular limitation on the method of purifying the compound of formula (I) obtained by the above reaction. Generally, after the reaction, the reaction mixture may be distilled under atmospheric or reduced pressure. As required, washing, recrystallization and chromatography may also be used for purification. When the compound (I) obtained has a high boiling point, lowboiling components such as the solvent are removed, and the residue is again dissolved in a solvent. The solution is washed with water and a dilute aqueous alkaline solution, and then the solvent is removed. As a result, the unreacted silane compound and carboxylic acid derivative, a by-product silane compound, etc. can be easily removed, and the desired compound of formula (I) can be obtained in pure form.

Specific examples of especially useful N,N-disubstituted carboxylic acid amides of formula (I) obtained by the process of this invention are given below. In the following compounds, Et represents an ethyl group.

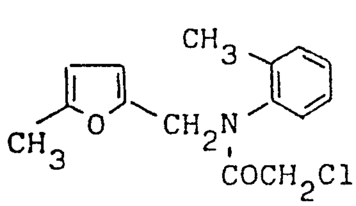 , 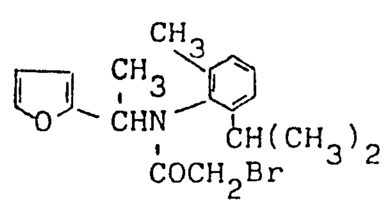 ,

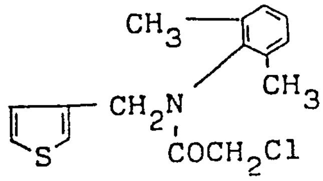 , 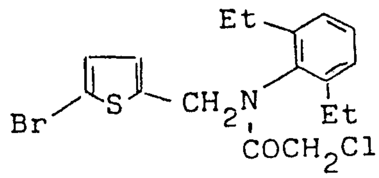 ,

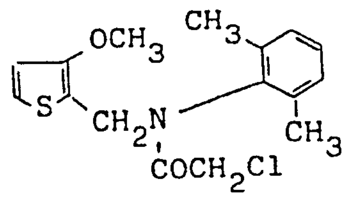 , 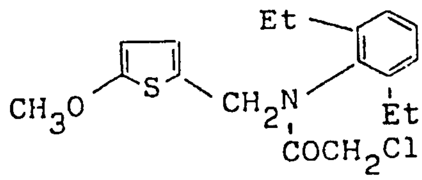 ,

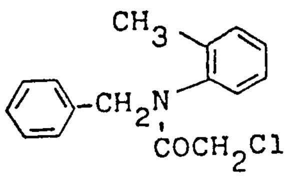 , 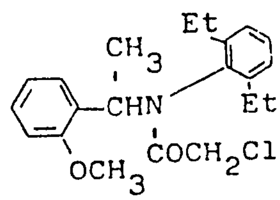 , 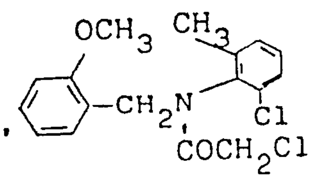

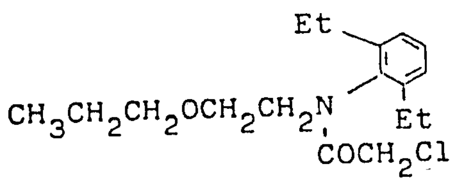 , 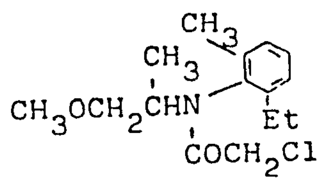 ,

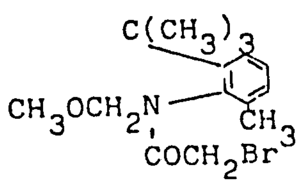 , 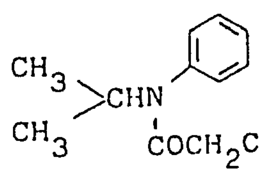 , 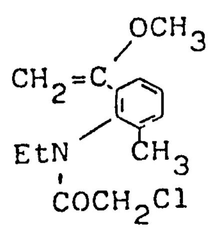 ,

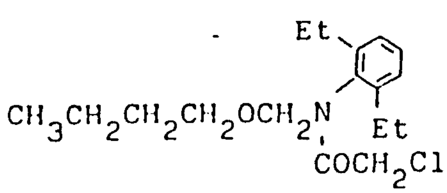 , 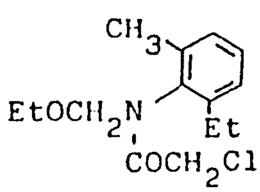 , 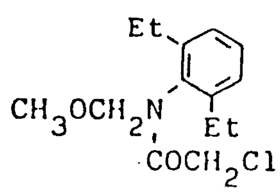 ,

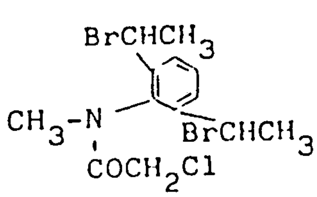 . , 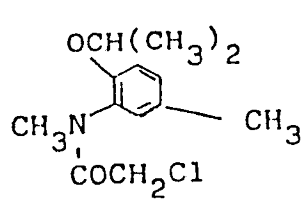 , 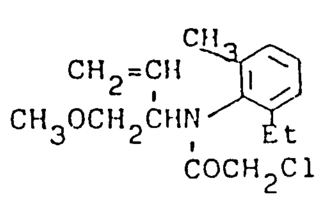 ,

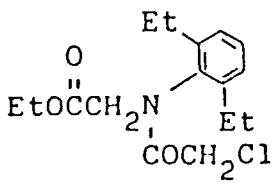 , 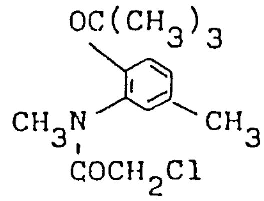 , 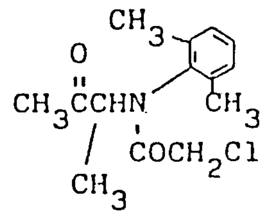 ,

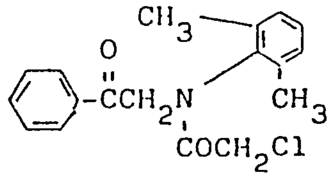 , 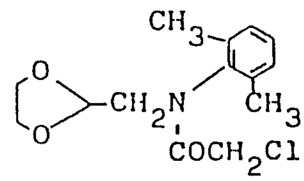 ,

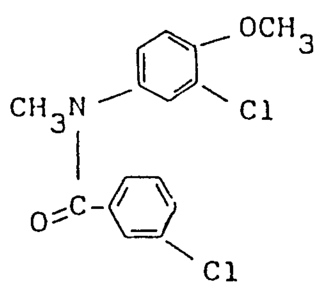 , 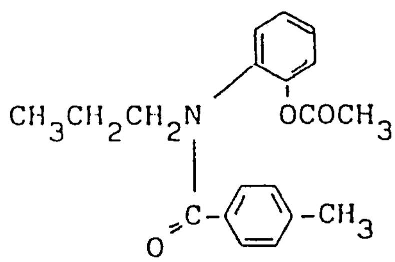 ,

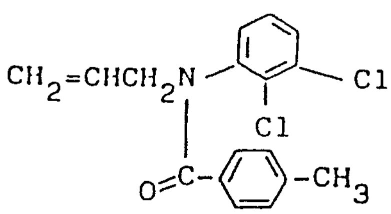 , 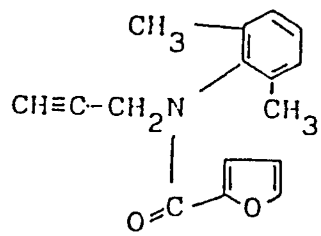 ,

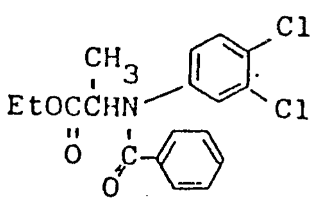 , 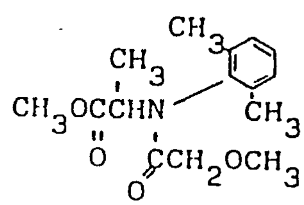 ,

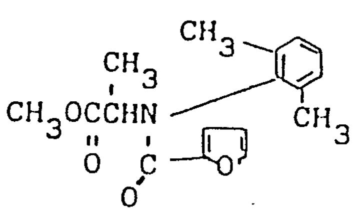 , 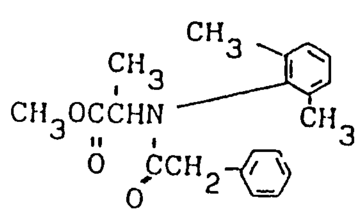 ,

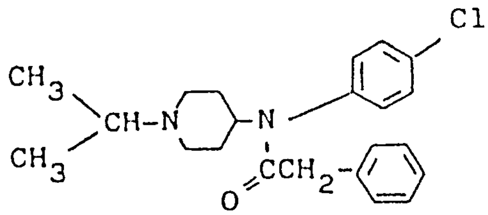 ,

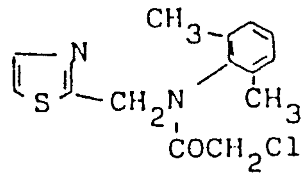 ,

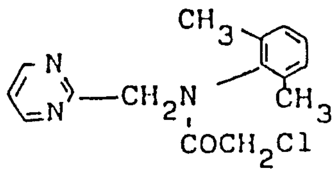 ,

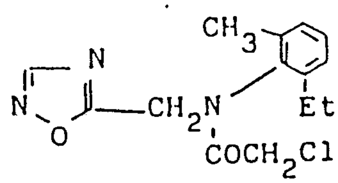 ,

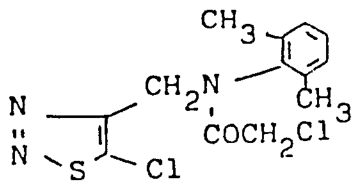 ,

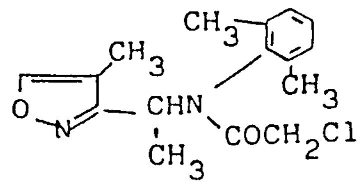 ,

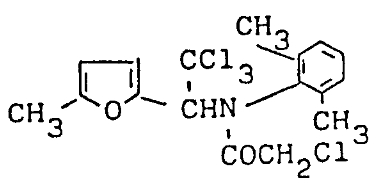 ,

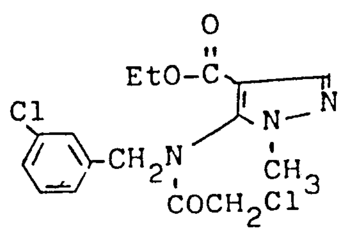 ,

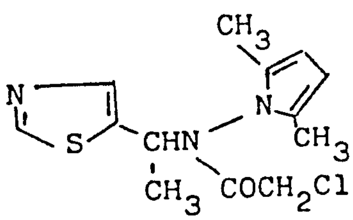 ,

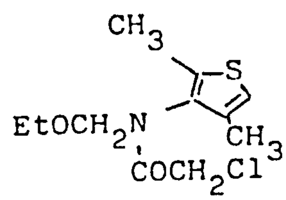 ,

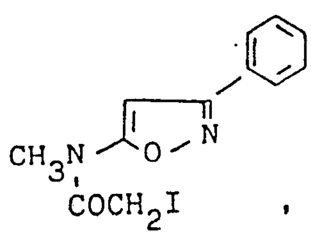 ,

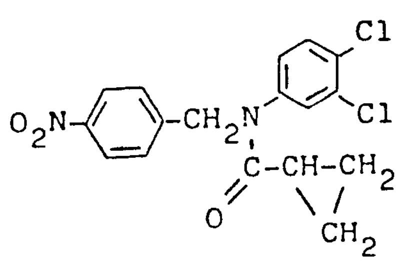 ,

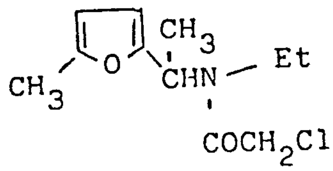 ,

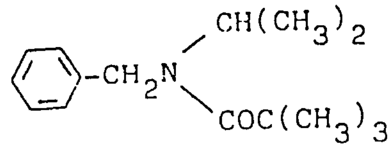 ,

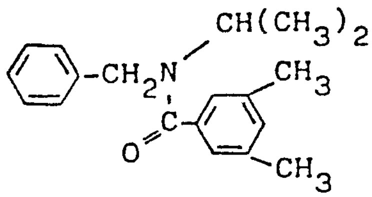 ,

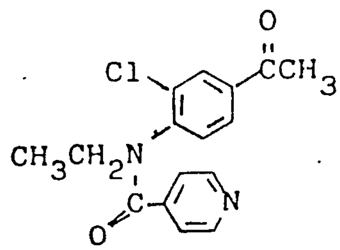 ,

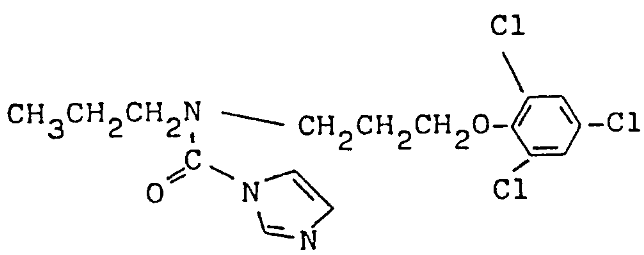 ,

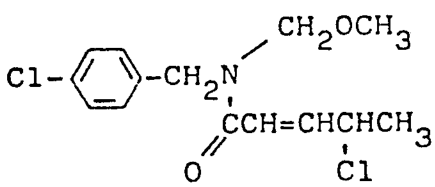 ,

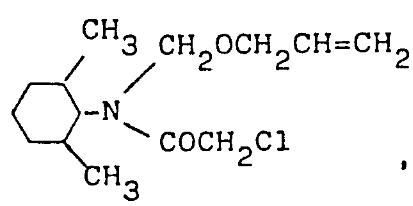 ,

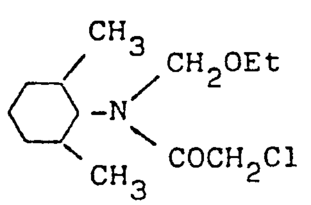 ,

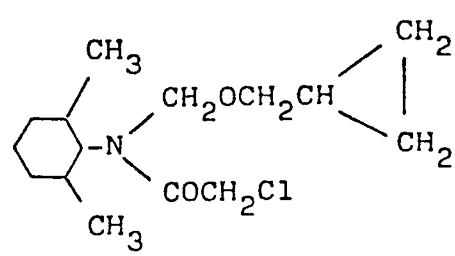 ,

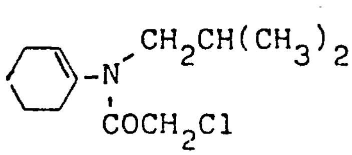 ,

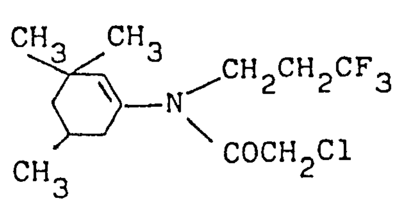 ,

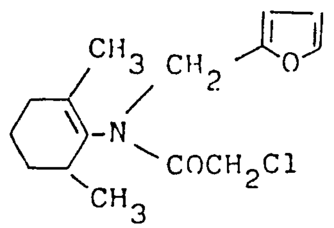 , 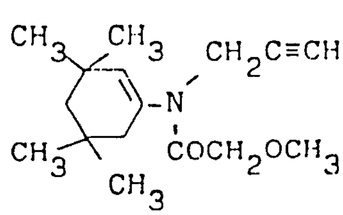 ,

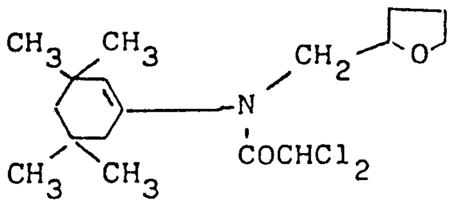 , 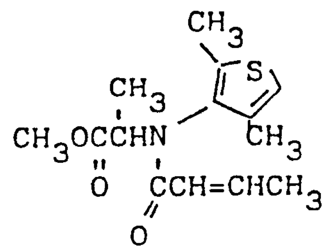

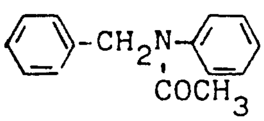 , 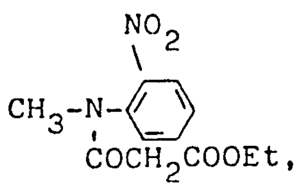 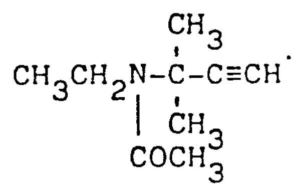 ,

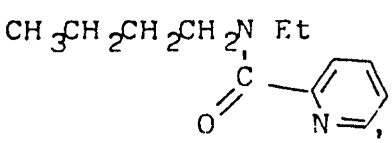 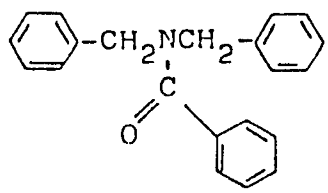 , 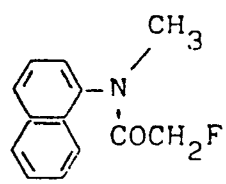 ,

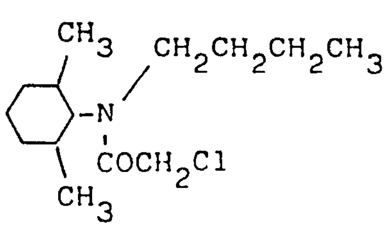 , 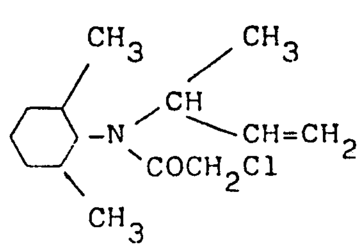

The N,N-disubstituted carboxylic acid amides obtained by the process of this invention are known to be useful as medicines and agricultural chemicals such as herbicides, insecticides or fungicides andintermediates for the production of the medicines and the agricultural chemicals. Accordingly, they can be used in these known applications without any restriction.

The process of this invention is an excellent process for producing N,N-disubstituted carboxylic acid amides useful as medicines and agricultural chemicals in good yields from the corresponding Schiff base compounds in one step under mild reaction conditions. After the reaction,

the resulting product can be easily purified. Accordingly, the present invention offers an excellent, industrial advantage.

The following Examples illustrate the present invention more specifically. It should be understood that the invention is in no way limited by these examples.

Example 1

A three-necked flask was charged with a solution of 2-(3-methoxy)-thienylmethylidene-2',6'-dimethylaniline (2.49 g) in dry benzene (15 ml), and under a nitrogen atmosphere, a solution of trichlorosilane (2.48 g) in dry benzene (5 ml) was gradually added dropwise with stirring at room temperature. Thereafter, a solution of chloroacetyl chloride (1.22 g) in dry benzene (5 ml) was gradually added, and the mixture was stirred at room temperature for 2 hours. Low-boiling components were removed, and the remaining viscous liquid was again dissolved in benzene (50 ml). The benzene solution was washed with water and then with a dilute aqueous alkaline solution, and dried over anhydrous sodium sulfate. Benzene was removed, and the resulting viscous liquid was dried under vacuum to give a pale brown solid (3.30 g). when distilled, this compound had a boiling point of 172 °C/0.15 mmHg.

The infrared absorption spectrum of this compound showed an absorption at 3100–2800 $cm^{-1}$ assigned to the C–H bond, and a strong absorption at 1670 $cm^{-1}$ assigned to the carbonyl linkage of the amide group.

Elemental analysis values were C 59.37%, H 5.89%, and N 4.05%, which well agreed with the calculated values for the composition formula $C_{16}H_{18}NSO_2Cl$ (323.84), i.e. C 59.33%, H 5.61% and N 4.33%.

The mass spectrum of the compound showed a molecular ion peak, $M^{\oplus}$, corresponding to the molecular weight at m/e 323, a peak corresponding to $M^{\oplus}$–Cl at m/e 288, a peak corresponding to $M^{\oplus}$–$COCH_2Cl$ at m/e 246, and a peak corresponding to 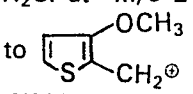 at m/e 127 (100%).

The $^1$H–NMR spectrum (δ ppm; internal standard tetramethylsilane; deuterochloroform solvent) of the compound was also measured. The results were as follows:

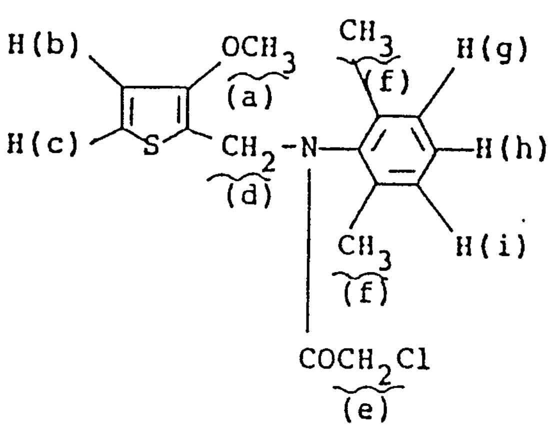

It shows a singlet at 1.95 ppm for 6 protons corresponding to the methyl protons of (f) substituted at the 2- and 6-positions of the phenyl group; a singlet at 3.50 ppm for 3 protons corresponding to the methyl group of (a); a singlet at 3.72 ppm for 2 protons corresponding to the methylene group of (d); a quadruplet at 6.55 ppm for 2 protons corresponding to the protons of the thiophene ring of (b) and (c); and a multiplet at 7.00–7.45 ppm for 3 group corresponding to the protons of the benzene ring at (g), (h) and (i).

The above results led to the determination that the isolated product was N-[2'-(3'-methoxy)-thienylmethyl]- N-chloroaceto-2,6-dimethylanilide. The yield was 93%.

Example 2

The same reaction as in Example 1 was carried out by using acetonitrile as the solvent. After the reaction, low-boiling components were removed, and the resulting viscous liquid was dried in vacuum to form a solid. Recrystallization of the solid from hexane gave N-[2'-(3'-methoxy)-thienylmethyl]- N-chloroaceto-2,6-dimethylanilide as white crystals in a yield of 55%.

Example 3

A solution of 2-(N-methylpyrrolyl)- methylidene-2',6'-diethylaniline (2.40 g) in dry benzene (20 ml) was put in a flask. Under a nitrogen atmosphere, a solution of chloroacetyl chloride (1.42 g) in dry benzene (5 ml) was gradually added with stirring at room temperature. Thereafter, a solution of trichlorosilane (2.38 g) in dry benzene (10 ml) was added. After the addition, the mixture was heated with stirring for 1 hour on an oil bath (50 °C). Low-boiling components were removed under reduced pressure, and the resulting viscous liquid was purified by column chromatography to give N'-[2-(N-methylpyrrolyl) methyl]-N'-chloroaceto-2',6'-diethylanilide of the following formula as a pale brown solid in a yield of 64%.

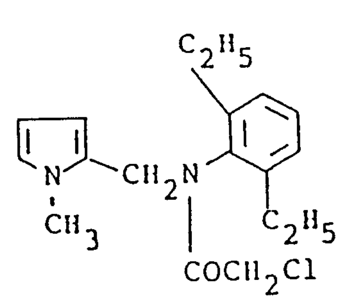

Example 4

A mixture of n-hexylaldehyde (1.53 g), 2,6-diethylaniline (2.24 g) and benzene (50 ml) was heated under reflux for 1 hour over an oil bath to perform azeotropic dehydration. To the resulting solution were added a solution of chloroacetyl chloride (1.79 g) in dry benzene (5 ml) and then a solution of trichlorosilane (3.40 g) in dry benzene (5 ml) with stirring at room temperature. After the addition, the mixture was heated

with stirring for 2 hours on an oil bath (40 °C). Low-boiling components were removed under reduced pressure, and the resulting viscous liquid was dissolved in ether (50 ml). The ether solution was washed with water, and dried over anhydrous sodium sulfate. Ether was removed, and the resulting liquid was distilled to isolate N-(n-hexyl)-N-chloroaceto- 2,6-diethylanilide of the following formula having a boiling point of 152 °C/ 0.2 mmHg in a yield of 55% based on 2,6-diethylaniline.

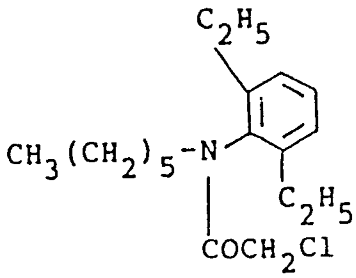

Example 5

In the same way as in Examples 1, 2 and 3, various amide compounds were synthesized using trichlorosilane as the silane compound. The structural formulae, properties and yields of the resulting N,N-disubstituted carboxylic acid amides are summarized in Table 1.

Table 1

| No. | $R^1-CH_2-N-R^2$<br>\|<br>$O-C-R^3$ | | | Property | Yield |
|---|---|---|---|---|---|
| | $R^1$ | $R^2$ | $R^3$ | | |
| 1 | $C_3H_7-$ | $-C_{10}H_{21}$ | $-CH_2C\ell$ | bp. 145°C/0.1 mmHg | 58 |
| 2 | 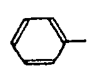 | 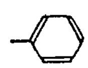 | $-CF_3$ | bp. 115°C/0.4 mmHg | 50 |
| 3 | 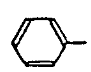 | 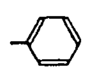 | 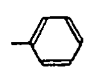 | mp. 95~7°C | 65 |
| 4 | 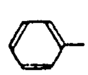 | 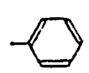 | $-C_5H_{11}$ | bp. 165°C/0.3 mmHg | 63 |
| 5 | $CH_3O-$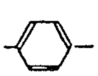 | $-CH_2CH_2OCH_3$ | $-CH_2C\ell$ | bp. 160°C/0.15 mmHg | 48 |
| 6 | 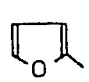 | 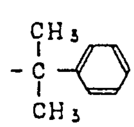 | $-CH_2C\ell$ | bp. 150°C/1.5 mmHg | 43 |

Table 1 (continued)

| No. | $R^1-CH_2-N(R^2)-O-C-R^3$ R¹ | R² | R³ | Property | Yield |
|---|---|---|---|---|---|
| 7 | 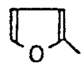 | $-CH_2CH_2OCH_3$ | $-CH_2Cl$ | bp 116℃/0.25mmHg | 51 |
| 8 | 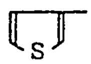 | 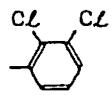 | $-CH_2Cl$ | bp.174℃/0.6mmHg | 53 |
| 9 | 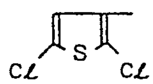 | 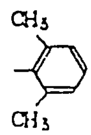 | $-CH_2Cl$ | bp.178℃/0.3mmHg | 40 |
| 10 | 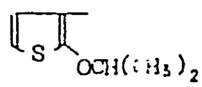 | 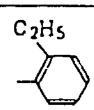 | $-CH_2Cl$ | bp.169℃/0.2mmHg | 38 |
| 11 | 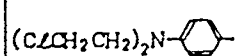 | 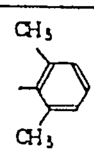 | $-CH_2Cl$ | mp. 83～5℃ | 45 |
| 12 | 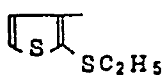 | 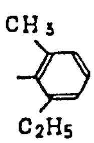 | $-CH_2Cl$ | bp.188℃/0.4mmHg | 60 |
| 13 | 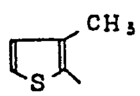 | 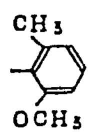 | $-CH_2Cl$ | bp.160℃/0.15mmHg | 54 |
| 14 | 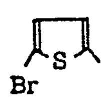 | 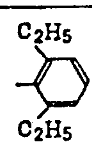 | $-CH_2Cl$ | bp.178℃/0.3mmHg | 47 |
| 15 | 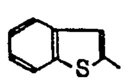 | 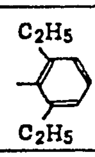 | $-CH_2Cl$ | bp.202℃/0.1mmHg | 55 |
| 16 | 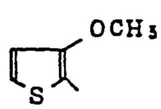 | 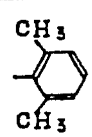 | $-CH_2OCH_3$ | bp.162℃/0.25mmHg | 73 |
| 17 | 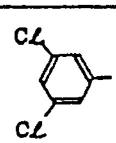 | 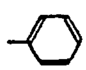 | $-CH_2Cl$ | bp.168℃/0.2mmHg | 96 |

Table 1 (continued)

| No. | 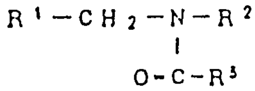 | | | Property | Yield |
|---|---|---|---|---|---|
| | R¹ | R² | R³ | | |
| 18 | 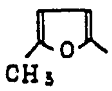 | 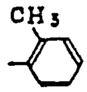 | 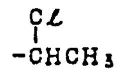 | bp.132°C/0.2 mmHg | 47 |
| 19 | 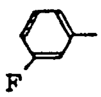 | 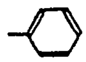 | $-CH_2Cl$ | bp.151°C/0.25 mmHg | 85 |
| 20 | 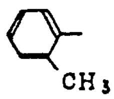 | 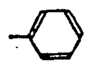 | $-CH_2Cl$ | bp.161°C/0.2 mmHg | 80 |
| 21 | 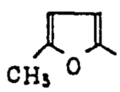 | 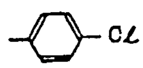 | $-CH_2Cl$ | bp.172°C/0.35 mmHg | 63 |
| 22 | 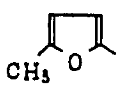 | 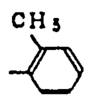 | $-CH_2CH_2Cl$ | bp.141°C/0.35 mmHg | 45 |
| 23 | 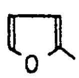 | 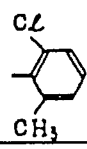 | $-CH_2Cl$ | bp.157°C/0.4 mmHg | 53 |
| 24 | 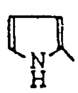 | 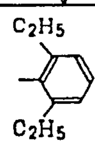 | $-CH_2Cl$ | bp.161°C/0.15 mmHg | 40 |
| 25 | 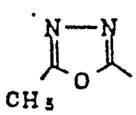 | 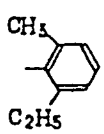 | $-CH_2Cl$ | mp.67-9°C | 80 |
| 26 | 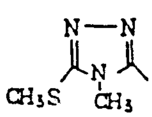 | 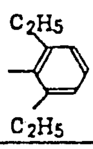 | $-CH_2Cl$ | mp.121-3°C | 75 |
| 27 | 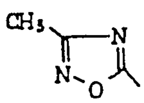 | 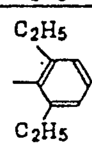 | $-CH_2Cl$ | mp.70-2°C | 84 |
| 28 | 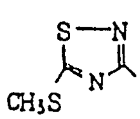 | 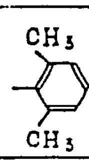 | $-CH_2Cl$ | mp. 55 | 71 |

Table 1 (continued)

| No. | $R^1-CH_2-N-R^2$ (N–C(=O)–$R^3$) | | | Property | Yield |
|---|---|---|---|---|---|
| | $R^1$ | $R^2$ | $R^3$ | | |
| 29 | 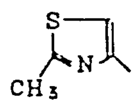 | 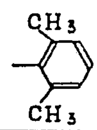 | $-CH_2Cl$ | mp. 60-2℃ | 78 |
| 30 | 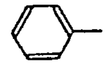 | 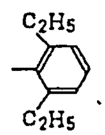 | $-CH_2Br$ | bp. 165~170℃/ 0.5mmHg | 90 |
| 31 | 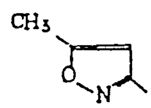 | 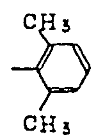 | $-CH_2Cl$ | mp. 73-6℃ | 85 |

Example 6

By the same method as described in Examples 1 to 4, N,N-disubstituted carboxylic acid amide compounds were synthesized by using various Schiff bases, silane compounds and carboxylic acid derivatives. The structural formulae and yields of the resulting amide compounds are summarized in Table 2.

In Table 2, $R^1$, $R^2$, $R^3$, M and HSiXYZ show the following.

$$R^1CH=N-R^2 \xrightarrow{R^3C(=O)M,\ HSiXYZ} R^1CH_2-N(-R^2)-C(=O)-R^3$$

Table 2

| No. | $R^1$ | $R^2$ | $R^3$ | M | HSiXYZ | Yield (%) |
|---|---|---|---|---|---|---|
| 1 | 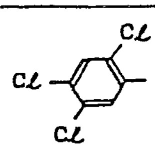 | $-CH_2CH(CH_3)_2$ | $-CH-CHCHCH_3$ (Cl) | Cl | $HSiCl_3$ | 41 |
| 2 | 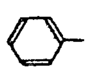 | 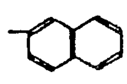 | $-CH_2Cl$ | Cl | $HSiCl_3$ | 55 |
| 3 | 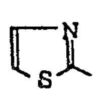 | 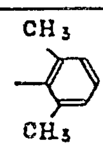 | $-CH_2Cl$ | Cl | $H_2SiCl_2$ | 60 |
| 4 | 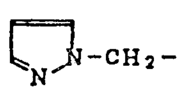 | 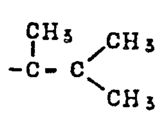 | $-CH_2Cl$ | Cl | $HSiCl_3$ | 39 |
| 5 | $CH_2-CH-$ | 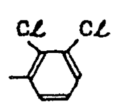 | 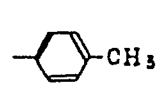 | Cl | $HSiCl_3$ | 45 |

Table 2 (continued)

| No | $R^1$ | $R^2$ | $R^3$ | M | HSiXYZ | Yield (%) |
|---|---|---|---|---|---|---|
| 6 | 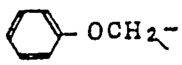 | 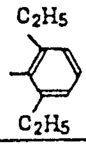 | $-CH_2Cl$ | Cl | $HSiCl_3$ | 50 |
| 7 | 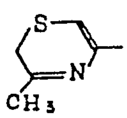 | 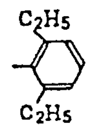 | $-CH_2Cl$ | Cl | $H_2SiCl_2$ | 48 |
| 8 | 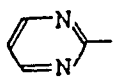 | 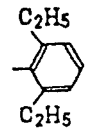 | $-CH_2Cl$ | Cl | $HSiCl_3$ | 48 |
| 9 | 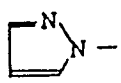 | 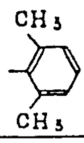 | 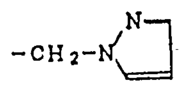 | Cl | $HSiCl_3$ | 54 |
| 10 | 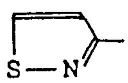 | 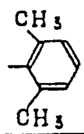 | $-CH_2Cl$ | Cl | $H_2SiCl_2$ | 60 |
| 11 | 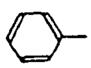 | $-CH(CH_3)_2$ | 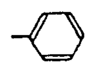 | Br | $HSiBr_3$ | 55 |
| 12 | $H_5C_2O\overset{O}{\overset{\|}{C}}CH_2-$ | 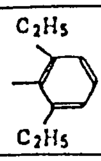 | $-CH_2Cl$ | Cl | $HSiCl_3$ | 42 |
| 13 | $CH_3-$ | $-C_2H_5$ | $-CH_2Cl$ | Cl | $HSiCl_3$ | 70 |
| 14 | $CH_2-CH-$ | $-CH_2CH-CH_2$ | $-CHCl_2$ | Cl | $H_2SiCl_2$ | 62 |
| 15 | 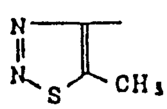 | 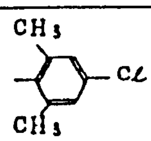 | $-CH_2Cl$ | Cl | $HSiCl_3$ | 38 |
| 16 | 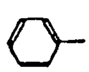 | $-CH(CH_3)_2$ | $-C(CH_3)_3$ | Cl | $H_2SiCl_2$ | 53 |

Table 2 (continued)

| No. | $R^1$ | $R^2$ | $R^3$ | M | HSiXYZ | Yield (%) |
|---|---|---|---|---|---|---|
| 17 | 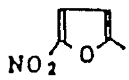 | $-(CH_2)_4-Cl$ | $-CH_2Br$ | Br | $HSiBr_3$ | 41 |
| 18 | 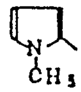 | $-CH_2-$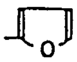 | $-CH_2Cl$ | $-OCCH_2Cl$ (C=O) | $HSiCl_3$ | 45 |
| 19 | $CH_2-CHCH_2-$ | 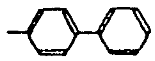 | $-CH_2CN$ | Cl | $HSiCl_3$ | 30 |
| 20 | 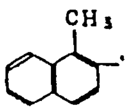 | $-CH_2C\equiv CH$ | $-CH_2CH_2NO_2$ | Br | $H_2SiBr_2$ | 29 |
| 21 | 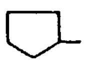 | $-(CH_2)_3SCH_3$ | 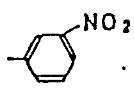 | Cl | $H_2SiCl_2$ | 45 |
| 22 | 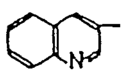 | 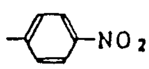 | $-CHCl_2$ | Cl | $HSiCl_3$ | 60 |
| 23 | 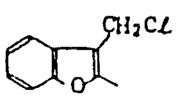 | 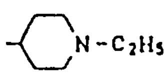 | $-CH_2CH_3$ | $-OCC_2H_5$ (C=O) | $H_3SiCl$ | 35 |
| 24 | 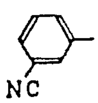 | 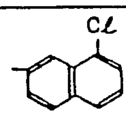 | $-CF_3$ | F | $HSiF_3$ | 28 |
| 25 | 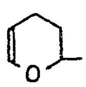 | 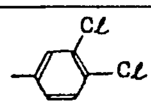 | $-CH_2I$ | Cl | $H_2SiCl_2$ | 35 |
| 26 | 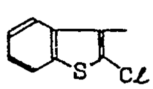 | $-(CH_2)_4CH-CH_2$ | $-CH_2-CH-$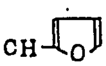 | Cl | $H_2SiCl_2$ | 29 |
| 27 | 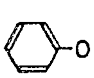$OCH_2CH_2-$ | 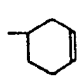 | $-CH_2CH(CH_3)OC_2H_5$ | Cl | $H_2SiCl_2$ | 38 |

Table 2 (continued)

| No | $R^1$ | $R^2$ | $R^3$ | M | HSiXYZ | Yield (%) |
|---|---|---|---|---|---|---|
| 28 | 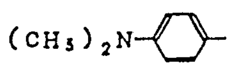 | 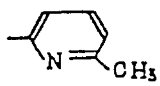 | $-CH_3$ | $-O\overset{O}{\overset{\|}{C}}CH_3$ | $HSiCl_3$ | 55 |
| 29 | 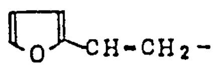 | 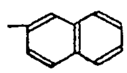 | $-CH_2Cl$ | Cl | $H_2SiCl_2$ | 40 |
| 30 | $CH_3-\overset{CH_3}{\underset{CH_3}{C}}-CH_2-$ | $-CH_2OC_2H_4$ | $-\overset{CF_3}{\underset{F}{C}}OC_3F_7$ | F | $HSiBr_3$ | 25 |
| 31 | $O_2N(CH_2)_{10}CH_2-$ | 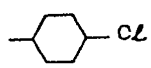 | 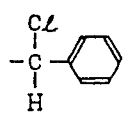 | Cl | $H_2SiCl_2$ | 38 |
| 32 | 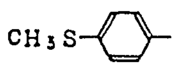 | 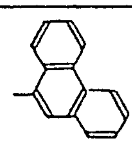 | $-CF_3$ | $-O\overset{O}{\overset{\|}{C}}CF_3$ | $HSiCl_3$ | 48 |
| 33 | 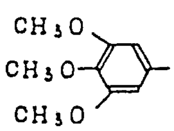 | 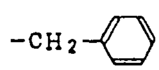 | $(CH_2)_4CN$ | Cl | $H_3SiCl$ | 65 |
| 34 | 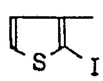 | 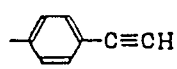 | $-CH_2OCH_3$ | Cl | $HSiCl_3$ | 27 |
| 35 | 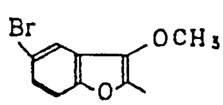 | $(CH_2)_9CH_3$ | $-\overset{Cl}{C}H-C_3H_7$ | Cl | $H_2SiCl_2$ | 33 |
| 36 | 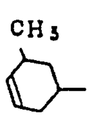 | 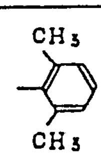 | $-CH_2CH_3$ | $-O\overset{O}{\overset{\|}{C}}C_2H_5$ | $HSCl_3$ | 45 |
| 37 | 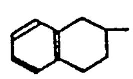 | 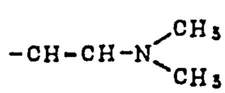 | 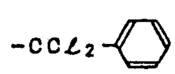 | Cl | $H_2SiCl_2$ | 34 |
| 38 | 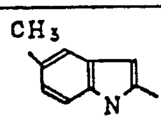 | 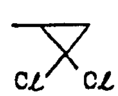 | $-CBr_2H$ | Br | $H_3SiBr$ | 28 |

Table 2 (continued)

| No. | $R^1$ | $R^2$ | $R^3$ | M | HSiXYZ | Yield (%) |
|---|---|---|---|---|---|---|
| 39 | $ClCH_2CH_2CH_2-$ | 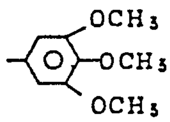 | 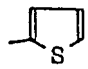 | Cl | $HSiCl_2$ | 47 |
| 40 | $CH_3OCH_2CH_2-$ | 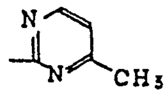 | $-CH_2COCH_3$ | Br | $H_2SiBr_2$ | 61 |
| 41 | 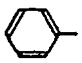 | 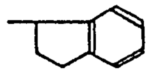 | 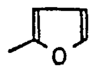 | Cl | $H_2SiCl_2$ | 55 |
| 42 | $CH_3{\equiv}CCH_2-$ | 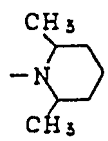 | 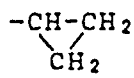 | Cl | $H_2SiCl_2$ | 43 |
| 43 | 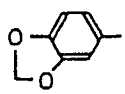 | 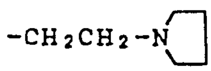 | 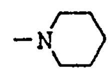 | Cl | $HSiCl_3$ | 70 |
| 44 | 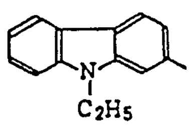 | $-CH_2CH_2OC_2H_5$ | 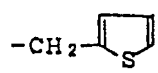 | Cl | $HSiCl_3$ | 68 |
| 45 | 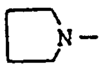 | 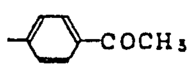 | $-CH_2CH_2SCH_3$ | Br | $HSiBr_3$ | 48 |
| 46 | 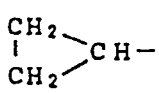 | 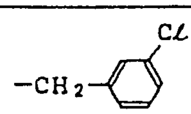 | 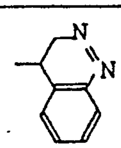 | Cl | $HSiCl_3$ | 39 |
| 47 | 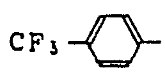 | $-CH_2(CF_2)_2CF_3$ | 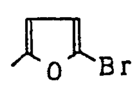 | Br | $H_2SiBr_2$ | 54 |
| 48 | 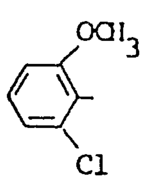 | 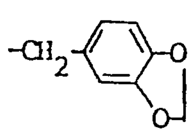 | 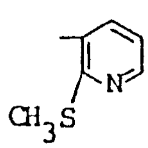 | Cl | $HSiCl_3$ | 41 |

Table 2 (continued)

| No | $R^1$ | $R^2$ | $R^3$ | M | HSiXYZ | Yield (%) |
|---|---|---|---|---|---|---|
| 49 | 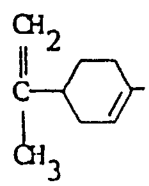 | 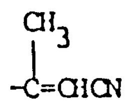 | $-CH_2Br$ | Br | $H_3SiBr$ | 53 |
| 50 | $NCCH_2CH_2-$ | 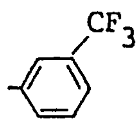 | 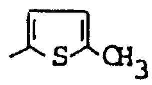 | Cl | $HSiCl_3$ | 57 |
| 51 | 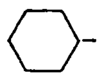 | 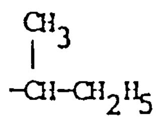 | $-CH_2Cl$ | Cl | $HSiCl_3$ | 65 |
| 52 | 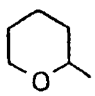 | $-C_4H_9$ | $-CH_2Cl$ | Cl | $H_2SiCl_2$ | 49 |
| 53 | 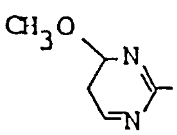 | 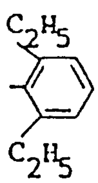 | $-CH_2Cl$ | Cl | $HSiCl_3$ | 53 |
| 54 | H | 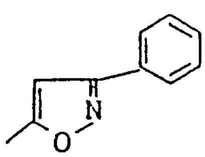 | $-CH_2I$ | Cl | $HSiCl_3$ | 58 |
| 55 | 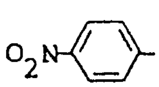 | 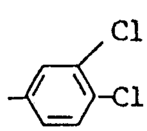 | 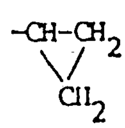 | Cl | $H_3SiCl$ | 72 |
| 56 | $CH_3-$ | 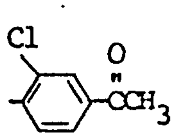 | 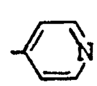 | Cl | $HSiCl_3$ | 82 |

Table 2 (continued)

| No. | $R^1$ | $R^2$ | $R^3$ | M | HSiXYZ | Yield (%) |
|---|---|---|---|---|---|---|
| 57 | $CH_3CH_2-$ | 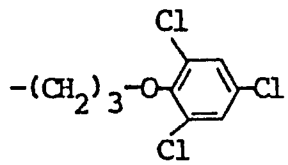 | 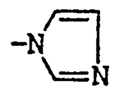 | Cl | $H_2SiCl_2$ | 78 |
| 58 | $(CH_3)_2CH-$ | 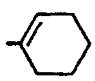 | $-CH_2Cl$ | Br | $HSiCl_3$ | 67 |
| 59 | $CF_3CH_2-$ | 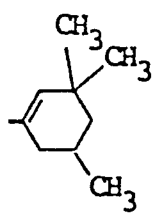 | $-CH_2Cl$ | Cl | $HSiCl_3$ | 74 |
| 60 | 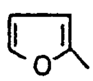 | 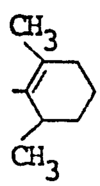 | $-CH_2Cl$ | Br | $H_2SiCl_2$ | 59 |
| 61 | $CH{\equiv}C-$ | 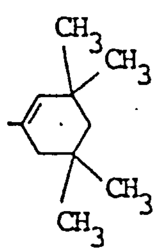 | $-CH_2OCH_3$ | Cl | $H_2SiCl_2$ | 75 |
| 62 | 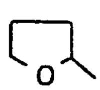 | 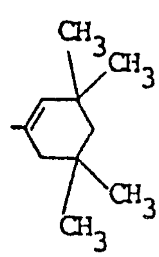 | $-CHCl_2$ | Cl | $HSiCl_3$ | 63 |
| 63 | 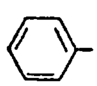 | 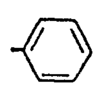 | $-CH_3$ | I | $H_3SiCl$ | 93 |

Table 2 (continued)

| No | R¹ | R² | R³ | M | HSiXYZ | Yield (%) |
|---|---|---|---|---|---|---|
| 64 | H | 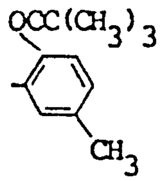 | $-CH_2Cl$ | $O\overset{O}{\overset{\|}{C}}CH_2Cl$ | $H_2SiCl_2$ | 87 |
| 65 | 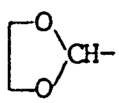 | 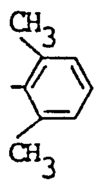 | $-CH_2Cl$ | Cl | $H_2SiCl_2$ | 70 |
| 66 | H | 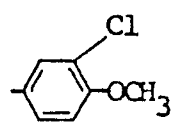 | 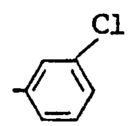 | Cl | $HSiCl_3$ | 92 |
| 67 | $CH_3CH_2-$ | 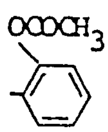 | 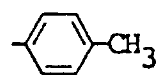 | Cl | $H_2SiCl_2$ | 89 |
| 68 | $CH_2=CH-$ | 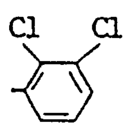 | 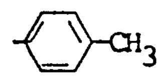 | Cl | $HSiCl_3$ | 78 |
| 69 | $CH\equiv C-$ | 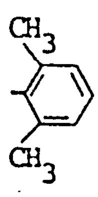 | 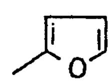 | Cl | $H_2SiCl_2$ | 74 |
| 70 | 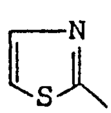 | 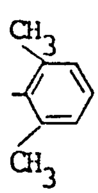 | $-CH_2Cl$ | Br | $HSiCl_3$ | 91 |

Table 2 (continued)

| No. | $R^1$ | $R^2$ | $R^3$ | M | HSiXYZ | Yield (%) |
|---|---|---|---|---|---|---|
| 71 | 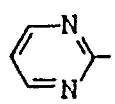 | 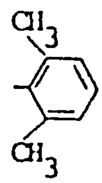 | $-CH_2Cl$ | Cl | $H_2SiCl_2$ | 75 |
| 72 | 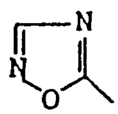 | 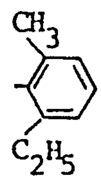 | $-CH_2Cl$ | Cl | $H_2SiCl_2$ | 63 |
| 73 | 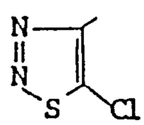 | 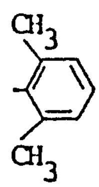 | $-CH_2Cl$ | Cl | $H_2SiCl_2$ | 59 |
| 74 | 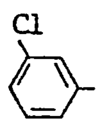 | 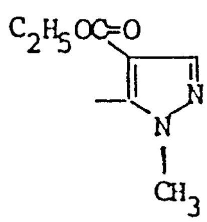 | $-CH_2Cl$ | Cl | $H_3SiBr$ | 68 |
| 75 | 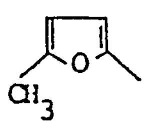 | 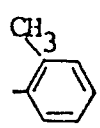 | $-CH_2Cl$ | $O\overset{O}{\overset{\|}{C}}CH_2Cl$ | $HSiCl_3$ | 94 |
| 76 | 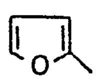 | 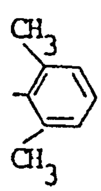 | $-CH_2Cl$ | Cl | $H_2SiCl_2$ | 82 |
| 77 | 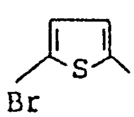 | 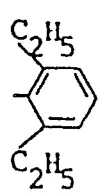 | $-CH_2Cl$ | F | $H_2SiCl_2$ | 70 |

Table 2 (continued)

| No. | $R^1$ | $R^2$ | $R^3$ | M | HSiXYZ | Yield (%) |
|---|---|---|---|---|---|---|
| 78 | 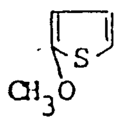 | 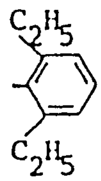 | $-CH_2Cl$ | Cl | $HSiCl_3$ | 91 |
| 79 | 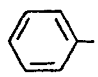 | 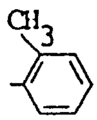 | $-CH_2Cl$ | Br | $HSiCl_3$ | 83 |
| 80 | 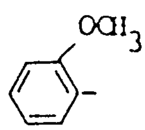 | 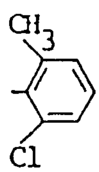 | $-CH_2Cl$ | Cl | $HSiCl_3$ | 95 |
| 81 | $CH_3CH_2-$<br>$CH_2OCH_2-$ | 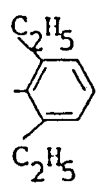 | $-CH_2Cl$ | Cl | $H_2SiCl_2$ | 88 |
| 82 | $CH_3$ | 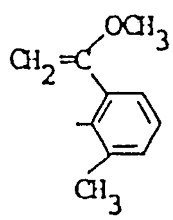 | $-CH_2Cl$ | $O\overset{O}{\overset{\|}{C}}CH_2Cl$ | $H_2SiCl_2$ | 51 |
| 83 | H | 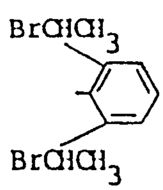 | $-CH_2Cl$ | Br | $HSiF_3$ | 67 |
| 84 | H | 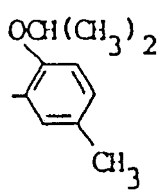 | $-CH_2Cl$ | Cl | $H_2SiCl_2$ | 96 |

Table 2 (continued)

| No. | $R^1$ | $R^2$ | $R^3$ | M | HSiXYZ | Yield (%) |
|---|---|---|---|---|---|---|
| 85 | H | 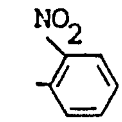 | 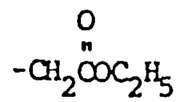 | Cl | $HSiCl_3$ | 86 |
| 86 | $CH_3-$ | 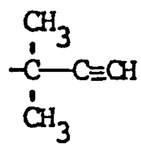 | $-CH_3$ | Br | $H_2SiBr_2$ | 73 |
| 87 | $CH_3CH_2CH_2-$ | $-C_2H_5$ | 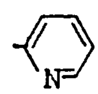 | Cl | $HSiCl_3$ | 68 |
| 88 | 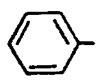 | 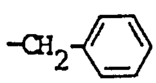 | 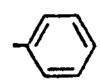 | Br | $HSiBr_3$ | 71 |
| 89 | H | 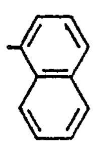 | $-CH_2F$ | Cl | $H_2SiCl_2$ | 59 |
| 90 | 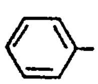 | $-CH(CH_3)_2$ | $-C(CH_3)_3$ | Cl | $HSiCl_3$ | 62 |
| 91 | 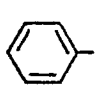 | $-CH(CH_3)_2$ | 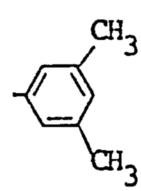 | Cl | $HSiCl_3$ | 84 |

Example 7

N,N-disubstituted carboxylic acid amide compounds were synthesized in the same way as in Example 6 except that Schiff base compounds of the following formula were used. The results are shown in Table 3.

In table 3, $R^1$, $R^2$, $R^3$, M and HSiXYZ show the following.

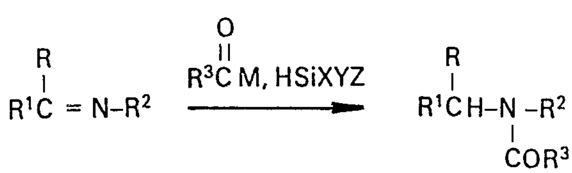

Table 3

| No. | R | $R_1$ | $R_2$ | $R_3$ | M | HSiXYZ | Yield (%) |
|---|---|---|---|---|---|---|---|
| 1 | $CH_3-$ | $CH_3OCH_2-$ | 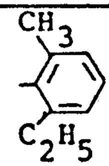 | $-CH_2Cl$ | $O\overset{O}{\overset{\|}{C}}CH_2Cl$ | $H_2SiCl_2$ | 66 |
| 2 | $CH_3-$ | $CH_3-$ | 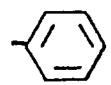 | $-CH_2Cl$ | Br | $H_2SiCl_2$ | 73 |
| 3 | $CH_2=CH-$ | $CH_3OCH_2-$ | 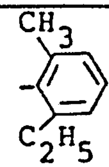 | $-CH_2Cl$ | F | $H_2SiF_2$ | 62 |
| 4 | $CH_3\overset{O}{\overset{\|}{C}}-$ | $CH_3-$ | 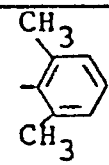 | $-CH_2Cl$ | Cl | $HSiCl_3$ | 67 |
| 5 | $C_2H_5O\overset{O}{\overset{\|}{C}}-$ | $CH_3-$ | 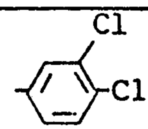 | 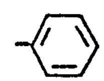 | 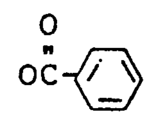 | $HSiCl_3$ | 59 |
| 6 | $CH_3O\overset{O}{\overset{\|}{C}}-$ | $CH_3-$ | 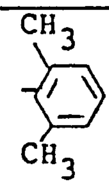 | $-CH_2OCH_3$ | Cl | $H_2SiCl_2$ | 75 |

Table 3 (continued)

| No. | R | $R_1$ | $R_2$ | $R_3$ | M | HSiXYZ | Yield (%) |
|---|---|---|---|---|---|---|---|
| 7 | $CH_3O\overset{O}{\overset{\|}{C}}-$ | $CH_3-$ | 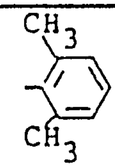 | 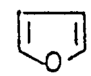 | Cl | $HSiCl_3$ | 63 |
| 8 | $CH_3O\overset{O}{\overset{\|}{C}}-$ | $CH_3-$ | 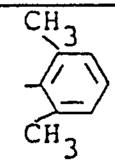 | $-CH_2-$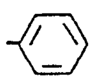 | Cl | $HSiCl_3$ | 80 |
| 9 | $CH_3-$ | 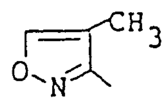 | 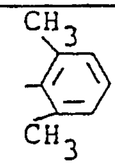 | $-CH_2Cl$ | $O\overset{O}{\overset{\|}{C}}CH_2Cl$ | $H_2SiCl_3$ | 64 |
| 10 | $CCl_3-$ | 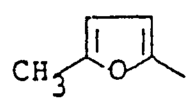 | 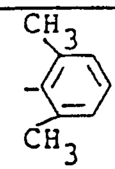 | $-CH_2Cl$ | Cl | $H_3SiCl$ | 58 |
| 11 | $CH_3-$ | 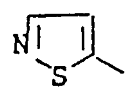 | 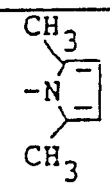 | $-CH_2Cl$ | Br | $HSiCl_3$ | 77 |
| 12 | $CH_3-$ | 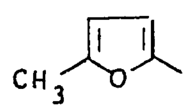 | $-C_2H_5$ | $-CH_2Cl$ | Cl | $HSiCl_3$ | 67 |

Table 3 (continued)

| No. | R | $R_1$ | $R_2$ | $R_3$ | M | HSiXYZ | Yield (%) |
|---|---|---|---|---|---|---|---|
| 13 | O<br>$CH_3OC-$ | $CH_3-$ | 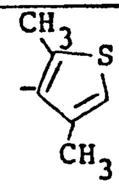 | $-CH=CHCH_3$ | Cl | $H_2SiCl_2$ | 56 |
| 14 | $CH_3O-$ | H | 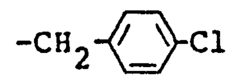 | $-CH=CHCHCH_3$<br>Cl | Cl | $HSiCl_3$ | 78 |
| 15 | $C_2H_5O-$ | H | 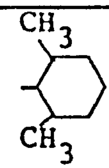 | $-CH_2Cl$ | Cl | $HSiCl_3$ | 85 |
| 16 | 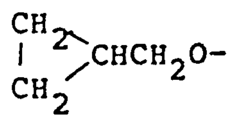 | H | 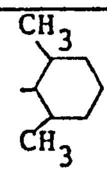 | $-CH_2Cl$ | I | $HSiCl_3$ | 66 |
| 17 | O<br>$C_2H_5OC-$ | H | 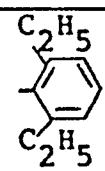 | $-CH_2Cl$ | Cl | $H_2SiCl_2$ | 80 |
| 18 | 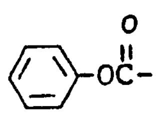 | H | 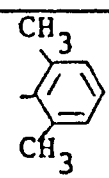 | $-CH_2Cl$ | Cl | $HSiCl_3$ | 93 |

Table 3 (continued)

| No. | R | $R_1$ | $R_2$ | $R_3$ | M | HSiXYZ | Yield (%) |
|---|---|---|---|---|---|---|---|
| 19 | $C_2H_5O-$ | H | 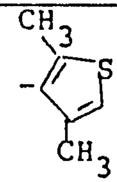 | $-CH_2Cl$ | Cl | $H_2SiCl_2$ | 88 |
| 20 | $CH_3O-$ | H | 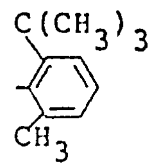 | $-CH_2Br$ | Br | $H_2SiBr_2$ | 62 |
| 21 | $CH_3CH_2CH_2CH_2O-$ | H | 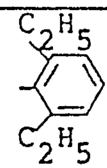 | $-CH_2Cl$ | Cl | $HSiCl_3$ | 81 |
| 22 | $C_2H_5O-$ | H | 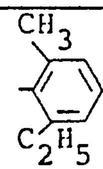 | $-CH_2Cl$ | $OC(=O)CH_2Cl$ | $H_2SiCl_2$ | 74 |
| 23 | $CH_3O-$ | H | 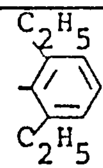 | $-CH_2Cl$ | Cl | $HSiCl_3$ | 89 |
| 24 | $CH_2=CHCH_2O-$ | H | 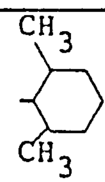 | $-CH_2Cl$ | Cl | $H_2SiCl_2$ | 57 |

Table 3 (continued)

| No. | R | $R_1$ | $R_2$ | $R_3$ | M | HSiXYZ | Yield (%) |
|---|---|---|---|---|---|---|---|
| 25 | $C_6H_{13}-$ | $C_6H_{13}-$ | $-C_4H_9$ | $-CH_2Cl$ | Cl | $HSiCl_3$ | 71 |
| 26 | $C_4H_9-$ | 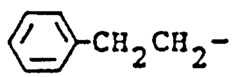 | 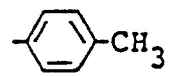 | $-CH_2Cl$ | Cl | $H_2SiCl_2$ | 74 |
| 27 | 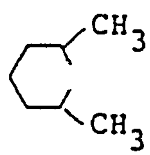 | | $-CH_2CH_2CH_2CH_3$ | $-CH_2Cl$ | F | $HSiF_3$ | 76 |
| 28 | 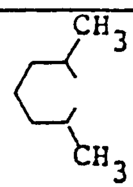 | | 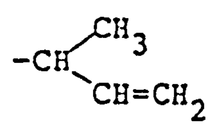 | $-CH_2Cl$ | Cl | $HSiCl_3$ | 73 |
| 29 | 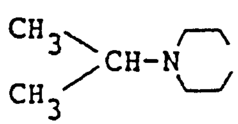 | | 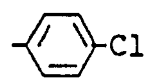 | 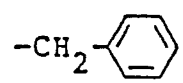 | Cl | $H_2SiCl_2$ | 66 |

**Claims**

1. A process for producing N,N-disubstituted carboxylic acid amides represented by the following formula (I)

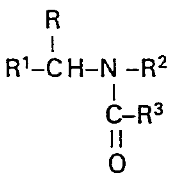
(I)

wherein R represents a hydrogen atom, an alkyl group, an alkenyl group, an alkoxy group, an alkenyloxy group, alkylcarbonyl groups having alkyl with 1 to 8 carbon atoms, alkoxycarbonyl groups having alkoxy with 1 to 8 carbon atoms, and arylcarbonyl groups having substituted or unsubstituted aryl having 6 to 10 carbon atoms, or a trihalogenomethyl group; $R^1$, $R^2$ and $R^3$, independently from each other, represent a substituted or unsubstituted alkyl group, a substituted or unsubstituted alkenyl group, a substituted or unsubstituted alkynyl group, a substituted or unsubstituted aryl group, a substituted or unsubstituted heteroaryl group, a substituted or unsubstituted cycloalkyl group, a substituted or unsubstituted cycloalkenyl group or a substituted or unsubstituted heterocycloalkyl group; $R^1$ may further represent a hydrogen atom; and R and $R^1$, taken together, may form a substituted or unsubstituted cyclic group together with the carbon atom to which they are bonded, which comprises contacting a Schiff base represented by the following formula (II)

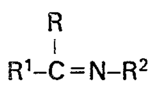
(II)

wherein R, $R^1$ and $R^2$ are as defined above, with a carboxylic acid derivative represented by the following formula (III)

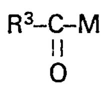
(III)

wherein $R^3$ is as defined above, and M represents a halogen atom or the moiety $-O\underset{\underset{O}{\|}}{C}R^3$, in the presence of a silane compound represented by the following formula (IV)

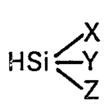
(IV)

wherein X, Y and Z, independently from each other, represent a hydrogen atom or a halogen atom, providing that at least one of X, Y and Z is halogen, in the presence or absence of an inert organic solvent.

2. The process of claim 1 wherein the contacting is carried out at a temperature in the range of −20 °C to 150 °C.

3. The process of claim 1 wherein the amount of the carboxylic acid derivative of formula (III) is 1 to 1.2 moles, and the amount of the silane compound of formula (IV) is 0.34 to 2.2 moles, both per mole of the Schiff base of formula (II).

4. The process of claim 1 wherein the Schiff base of formula (II) is selected from the group consisting of compounds of the following formula

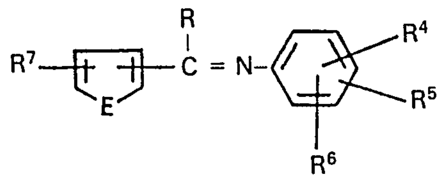
(II-1)

wherein E represents O or S, R is as defined above, $R^4$, $R^5$, $R^6$ and $R^7$, independently from each other, are the same as defined above for $R^1$, compounds of the following formula

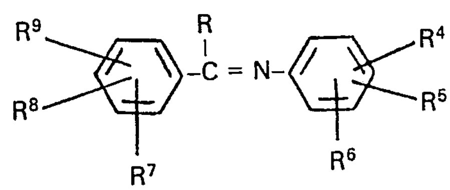
(II-2)

wherein R is as defined above, and $R^4$, $R^5$, $R^6$, $R^7$, $R^8$ and $R^9$, independently from each other, are the same as defined above for $R^1$, compounds of the following formula

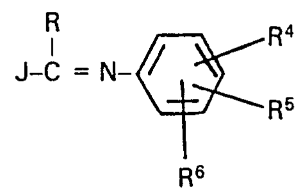
(II-3)

wherein J represents an alkyl group, an alkoxy group, an alkoxyalkyl group or an alkoxycarbonyl group, R is the same as defined above, and $R^4$, $R^5$ and $R^6$, independently from each other, are the same as defined above for $R^1$.

5. The process of claim 1 wherein the carboxylic acid derivative of formula (III) is selected from the group consisting of compounds of the formula

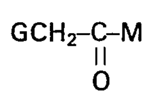
(III-1)

wherein M is as defined above, and G represents a hydrogen atom, a halogen atom, an alkyl group or an alkoxy group.

Compounds of the formula

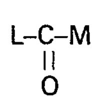
(III-2)

wherein M is as defined, and L represents a phenyl group, a pyridyl group, a furyl group or a benzyl group.

6. The process of claim 1 wherein R is a member selected from the class consistinq of a hydrogen atom, alkyl groups having 1 to 10 carbon atoms, alkenyl groups having 2 to 6 carbon atoms, alkoxy groups having 1 to 6 carbon atoms, alkenyloxy groups having 2 to 6 carbon atoms, alkylcarbonyl qroups having 1 to 8 carbon atoms in the alkyl moiety, alkoxycarbonyl groups having 1 to 8 carbon atoms in the alkoxy moiety, arylcarbonyl groups having substituted or unsubstituted $C_6$–$C_{10}$ aryl, said substituent for aryl being selected from alkyl groups having 1 to 6 carbon atoms, alkenyl groups having 2 to 6 carbon atoms, alkylnyl groups having 2 to 6 carbon atoms, alkoxy groups having 1 to 6 carbon atoms, alkylthio groups having 1 to 6 carbon atoms, alkoxycarbonyl groups having 1 to 4 carbon atoms in the alkoxy moiety, alkylcarbonyl groups having 1 to 4 carbon atoms in the alkyl moiety, halogen atoms, a cyano group, a nitro group, dialkylamino groups having 1 to 4 carbon atoms in each alkyl moiety, trihalogenomethyl groups and alkylcarbonyloxy groups having 1 to 4 carbon atoms in the alkyl moiety, and trihalogenomethyl groups in which the halogen is selected from Cl, Br and F.

7. The process of claim 1 wherein each of $R^1$, $R^2$ and $R^3$ is a member selected from the class consisting of substituted or unsubstituted alkyl groups having 1 to 15 carbon atoms, substituted or unsubstituted alkenyl groups having 2 to 15 carbon atoms, substituted or unsubstituted alkynyl groups having 2 to 15 carbon atoms, substituted or unsubstituted aryl groups having 6 to 20 carbon atoms, substituted or unsubstituted heteroaryl groups having 2 to 20 carbon atoms and 1 to 4 hetero atoms selected from N, O and S, substituted or unsubstituted cycloalkyl groups having 3 to 20 carbon atoms, substituted or unsubstituted cycloalkenyl groups having 3 to 20 carbon atoms and substituted or unsubstituted heterocycloalkyl groups having 2 to 20 carbon atoms and 1 to 4 hetero atoms selected from N, O and S; $R^1$ may further represent a hydrogen atom; R and $R^1$, taken together, may form a substituted or unsubstituted $C_4$–$C_{15}$ cycloalkyl group, or a heterocycloalkyl group, together with the carbon atom to which they are bonded; and the substituents in said substituted groups are selected from alkyl groups having 1 to 6 carbon atoms, alkenyl groups having 2 to 6 carbon atoms; alkynyl groups having 2 to 6 carbon atoms, alkoxy groups having 1 to 6 carbon atoms, alkylthio groups having 1 to 6 carbon atoms, alkoxycarbonyl groups having 1 to 4 carbon atoms in the alkoxy moiety, alkylcarbonyl groups having 1 to 4 carbon atoms in the alkyl moiety, halogen atoms, a cyano group, a nitro group, dialkylamino groups having 1 to 4 carbon atoms in each alkyl moiety, trihalogenomethyl groups and alkylcarbonyloxy groups having 1 to 4 carbon atoms in the alkyl moiety.

## Patentansprüche

1. Verfahren zur Herstellung von N,N-disubstituierten Carbonsäureamiden der folgenden allgemeinen Formel (I)

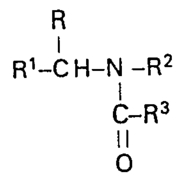

(I)

worin R ein Wasserstoffatom, eine Alkylgruppe, eine Alkenylgruppe, eine Alkoxygruppe, eine Alkenyloxygruppe, Alkylcarbonylgruppen mit Alkyl mit 1 bis 8 Kohlenstoffatomen, Alkoxycarbonylgruppen mit Alkoxy mit 1 bis 8 Kohlenstoffatomen, und Arylcarbonylgruppen mit substituiertem oder unsubstituiertem Aryl mit 6 bis 10 Kohlenstoffatomen oder eine Trihalogenmethylgruppe bedeutet; $R^1$, $R^2$ und $R^3$ unabhängig voneinander eine substituierte oder unsubstituierte Alkylgruppe, eine substituierte oder unsubstituierte Alkenylgruppe, eine substituierte oder unsubstituierte Alkinylgruppe, eine substituierte oder unsubstituierte Arylgruppe, eine substituierte oder unsubstituierte Heteroarylgruppe, eine substituierte oder unsubstituierte Cycloalkylgruppe, eine substituierte oder unsubstituierte Cycloalkenylgruppe oder eine substituierte oder unsubstituierte Heterocycloalkylgruppe bedeuten; $R^1$ weiter ein Wasserstoffatom bedeutet und R und $R^1$ zusammen eine substituierte oder unsubstituierte cyclische Gruppe zusammen mit dem Kohlenstoffatom, an das sie gebunden sind, bedeuten, dadurch gekennzeichnet, dass eine Schiffsche Base der folgenden Formel (II)

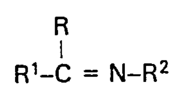

(II)

worin R, $R^1$ und $R^2$ die oben gegebene Definition besitzen, mit einem Carbonsäurederivat der folgenden Formel (III)

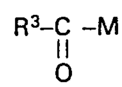

(III)

worin $R^3$ die oben gegebene Definition besitzt und M ein Halogenatom oder den Molekülteil $-OCR^3$ bedeutet, in Anwesenheit einer Silanverbindung der folgenden Formel (IV)

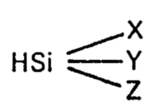

(IV)

worin X, Y und Z unabhängig voneinander ein Wasserstoffatom oder ein Halogenatom bedeuten, mit der Massgabe, dass mindestens eines von X, Y und Z ein Halogenatom bedeutet, in Anwesenheit oder Abwesenheit eines inerten organischen Lösungsmittels umgesetzt wird.

2. Verfahren nach Anspruch 1, dadurch gekennzeichnet, dass die Behandlung bei einer Temperatur im Bereich von −20 °C bis 150 °C durchgeführt wird.

3. Verfahren nach Anspruch 1, dadurch gekennzeichnet, dass die Menge des Carbonsäurederivats der Formel (III) 1 bis 1,2 Mol beträgt und dass die Menge der Silanverbindung der Formel (IV) 0,34 bis 2,2 Mol beträgt, beides pro Mol Schiff'scher Base der Formel (II).

4. Verfahren nach Anspruch 1, dadurch gekennzeichnet, dass die Schiff'sche Base der Formel (II) ausgewählt wird aus der Gruppe, die besteht aus Verbindungen der folgenden Formel

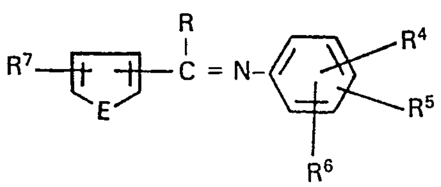

(II-1)

worin E, O oder S bedeutet, R die oben gegebene Definition besitzt, $R^4$, $R^5$, $R^6$ und $R^7$ unabhängig voneinander die gleichen Definitionen besitzen, wie sie oben für $R^1$ angegeben wurden, Verbindungen der folgenden Formel

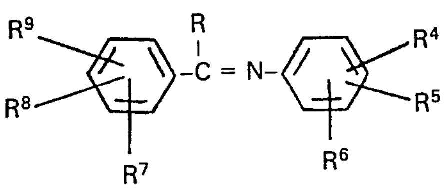

(II-2)

worin R die oben gegebene Definition besitzt und $R^4$, $R^5$, $R^6$, $R^7$, $R^8$ und $R^9$ unabhängig voneinander die gleichen Definitionen besitzen, wie sie oben für $R^1$ angegeben wurden, Verbindungen der folgenden Formel

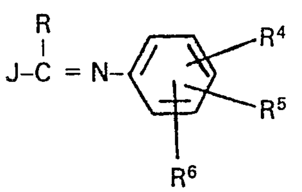

(II-3)

worin J eine Alkylgruppe, eine Alkoxygruppe, eine Alkoxyalkylgruppe oder eine Alkoxycarbonylgruppe bedeutet, R die gleiche Definition besitzt, wie sie oben angegeben wurde, und $R^4$, $R^5$ und $R^6$ unabhängig voneinander die gleichen Definitionen besitzen, wie sie oben für $R^1$ angegeben wurden.

5. Verfahren nach Anspruch 1, dadurch gekennzeichnet, dass das Carbonsäurederivat der Formel (III) ausgewählt wird aus der Gruppe, die besteht aus Verbindungen der Formel

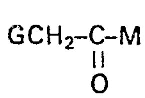

(III-1)

worin M die oben gegebene Definition besitzt und G ein Wasserstoffatom, ein Halogenatom, eine Alkylgruppe oder eine Alkoxygruppe bedeutet, Verbindungen der Formel

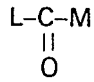

(III-2)

worin M die gegebene Definition besitzt, L eine Phenylgruppe, eine Pyridylgruppe, eine Furylgruppe oder eine Benzylgruppe bedeutet.

6. Verfahren nach Anspruch 1, dadurch gekennzeichnet, dass R ein Mitglied der Klasse ist, die besteht aus einem Wasserstoffatom, Alkylgruppen mit 1 bis 10 Kohlenstoffatomen, Alkenylgruppen mit 2 bis 6 Kohlenstoffatomen, Alkoxygruppen mit 1 bis 6 Kohlenstoffatomen, Alkenyloxygruppen mit 2 bis 6 Kohlenstoffatomen, Alkylcarbonylgruppen mit 1 bis 8 Kohlenstoffatomen in dem Alkylmolekülteil, Alkoxycarbonylgruppen mit 1 bis 8 Kohlenstoffatomen in dem Alkoxymolekülteil, Arylcarbonylgruppen mit substituiertem oder unsubstituiertem $C_6$–$C_{10}$-Aryl, wobei der Substituent für Aryl ausgewählt wird unter Alkylgruppen mit 1 bis 6 Kohlenstoffatomen, Alkenylgruppen mit 2 bis 6 Kohlenstoffatomen, Alkinylgruppen mit 2 bis 6 Kohlenstoffatomen, Alkoxygruppen mit 1 bis 6 Kohlenstoffatomen, Alkylthiogruppen mit 1 bis 6 Kohlenstoffatomen, Alkoxycarbonylgruppen mit 1 bis 4 Kohlenstoffatomen in dem Alkylmolekülteil, Alkylcarbonylgruppen mit 1 bis 4 Kohlenstoffatomen in dem Alkylmolekülteil, Halogenatomen, einer Cyanogruppe, einer Nitrogruppe, Dialkylaminogruppen mit 1 bis 4 Kohlenstoffatomen in jedem Alkylmolekülteil, Trihalogenmethylgruppen und Alkylcarbonyloxygruppen mit 1 bis 4 Kohlenstoffatomen in dem Alkylmolekülteil und Trihalogenmethylgruppen, wobei das Halogen ausgewählt wird unter Cl, Br und F.

7. Verfahren nach Anspruch 1, dadurch gekennzeichnet, dass jeder von $R^1$, $R^2$ und $R^3$ ein Mitglied, ausgewählt aus der Klasse ist, die besteht aus substituierten oder unsubstituierten Alkylgruppen mit 1 bis 15 Kohlenstoffatomen, substituierten oder unsubstituierten Alkenylgruppen mit 2 bis 15 Kohlenstoffatomen, substituierten oder unsubstituierten Alkinylgruppen mit 2 bis 15 Kohlenstoffatomen, substituierten oder unsubstituierten Arylgruppen mit 6 bis 20 Kohlenstoffatomen, substituierten oder unsubstituierten Heteroarylgruppen mit 2 bis 20 Kohlenstoffatomen und 1 bis 4 Heteroatomen, ausgewählt unter N, O und S, substituierten oder unsubstituierten Cycloalkylgruppen mit 3 bis 20 Kohlenstoffatomen, substituierten oder unsubstituierten Cycloalkenylgruppen mit 3 bis 20 Kohlenstoffatomen und substituierten oder unsubstituierten Heterocycloalkylgruppen mit 2 bis 20 Kohlenstoffatomen und 1 bis 4 Heteroatomen, ausgewählt unter N, O und S; $R^1$ weiter ein Wasserstoffatom bedeuten kann; R und $R^1$ zusammen eine substituierte oder unsubstituierte $C_4$–$C_{15}$-Cycloalkylgruppe oder eine Heterocycloalkylgruppe zusammen mit dem Kohlenstoffatom, an

das sie gebunden sind, bilden; und wobei die Substituenten in den substituierten Gruppen ausgewählt werden können unter Alkylgruppen mit 1 bis 6 Kohlenstoffatomen, Alkenylgruppen mit 2 bis 6 Kohlenstoffatomen, Alkinylgruppen mit 2 bis 6 Kohlenstoffatomen, Alkoxygruppen mit 1 bis 6 Kohlenstoffatomen, Alkylthiogruppen mit 1 bis 6 Kohlenstoffatomen, Alkoxycarbonylgruppen mit 1 bis 4 Kohlenstoffatomen in dem Alkoxymolekülteil, Alkylcarbonylgruppen mit 1 bis 4 Kohlenstoffatomen in dem Alkylmolekülteil, Halogenatomen, einer Cyanogruppe, einer Nitrogruppe, Dialkylaminogruppen mit 1 bis 4 Kohlenstoffatomen in jedem Alkylmolekülteil, Trihalogenmethylgruppen und Alkylcarbonyloxygruppen mit 1 bis 4 Kohlenstoffatomen in dem Alkylmolekülteil.

**Revendications**

1. Procédé de préparation de carboxamides N,N-disubstitués représentés par la formule suivante (I):

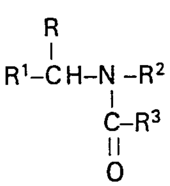

(I)

où R représente un atom d'hydrogène, un groupe alkyle, un groupe alkényle, un groupe alkoxy, un groupe alkényloxy, des groupes alkylcarbonyles ayant un alkyle avec 1 à 8 atomes de carbone, des groupes alkoxy carbonyles ayant un alkoxy avec 1 à 8 atomes de carbone, et des groupes aryles carbonyles ayant un aryle substitués ou non substitués avec 6 à 10 atomes de carbone ou un groupe trihalogénométhyle; $R^1$, $R^2$ et $R^3$, indépendamment l'un de l'autre, représentent un groupe alkyle substitué ou non substitué, un groupe alkényle substitué ou non substitué, un groupe alkynyle substitué ou non substitué, un groupe aryle substitué ou non substitué, un groupe hétéroaryle substitué ou non substitué, un groupe cycloalkyle substitué ou non substitué, un groupe cycloalkényle substitué ou non substitué, ou un groupe hétérocycloalkyle substitué ou non substitué; $R^1$ peut représenter en outre un atome d'hydrogène, et R et $R^1$ (pris en conjonction), peuvent former un groupe cyclique substitué ou non substitué, en conjonction avec l'atome de carbone auquel ils sont liés, procédé selon lequel on met en contact une base de Schiff représentée par la formule suivante (II):

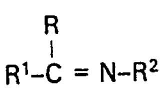

(II)

où R, $R^1$ et $R^2$ sont tels que définis plus haut, avec un dérivé d'acide carboxylique représenté par la formule suivante (III):

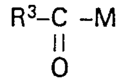

(III)

où $R^3$ est tel que défini ci-dessus, et M représente un atome d'halogène ou le radical 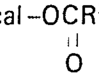, en présence d'un silane représenté par la formule suivante (IV):

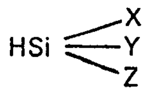

(IV)

où X, Y et Z, indépendamment les uns des autres, représentent un atome d'hydrogène ou un atome d'halogène, pourvu qu'au moins 1 de X, Y et Z soit un halogène, en présence ou en l'absence d'un solvant organique inerte.

2. Procédé selon la revendication 1, dans lequel la mise en contact est effectuée à une température dans la gamme de −20 °C à 150 °C.

3. Procédé selon la revendication 1, dans lequel la quantité du dérivé d'acide carboxylique de formule (III) va de 1 à 1,2 moles, et la quantité du silane de formule (IV) va de 0,34 à 2,2 moles, tous deux par mole de la base de Schiff de formule (II).

4. Procédé selon la revendication 1, dans lequel la base de Schiff de formule (II) est choisie dans le groupe constitué:
de composés de formule suivante:

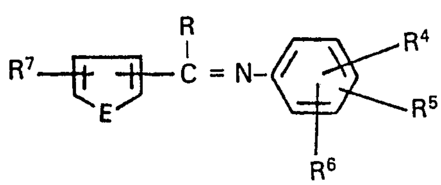

(II-1)

où E représente O ou S, R est tel que défini plus haut, $R^4$, $R^5$, $R^6$ et $R^7$, indépendamment les uns des autres, sont les mêmes que définis plus haut pour $R^1$, de composés de formule suivante:

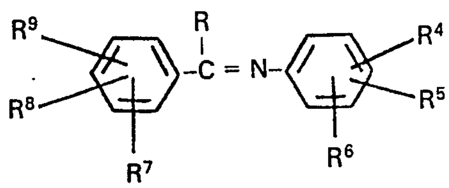

(II-2)

où R est tel que défini plus haut, et $R^4$, $R^5$, $R^6$ et $R^7$, $R^8$ et $R^9$, indépendamment les uns des autres, sont les mêmes que définis plus haut pour $R^1$, de composés de formule suivante:

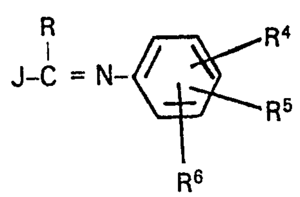

(II-3)

où J représente un groupe alkyle, un groupe alkoxy, un groupe alkoxyalkyle ou un groupe alkoxycarbonyle, R est le même que défini ci-dessus et $R^4$, $R^5$, et $R^6$, indépendamment les uns des autres, sont les mêmes que définis plus haut pour $R^1$.

5. Procédé selon la revendication 1, dans lequel le dérivé d'acide carboxylique de formule (III) est choisi dans le groupe constitué de composés de formule:

$GCH_2$–C–M (III-1)
 ||
 O

où M est tel que défini ci-dessus, et G représente un atome d'hydrogène, un atome d'halogène, un groupe alkyle ou un groupe alkoxy, de composés de formule:

L–C–M (III-2)
 ||
 O

où M est tel que défini, et L représente un groupe phényle, un groupe pyridyle, un groupe furyle ou un groupe benzyle.

6. Procédé selon la revendication 1, dans lequel R est un élément pris dans l'ensemble constitué par l'atome d'hydrogène, les alkyles contenant de 1 à 10 atomes de carbone, les alcényles contenant de 2 à 6 atomes de carbone, les alcoxy contenant de 1 à 6 atomes de carbone, les alcényloxy contenant de 2 à 6 atomes de carbone, les alkylcarbonyles contenant de 1 à 8 atomes de carbone dans la partie alkyle, les alcoxycarbonyles contenant de 1 à 8 atomes de carbone dans la partie alcoxy, les arylcarbonyles dont l'aryle renferme de 6 à 10 atomes de carbone et porte ou non des substituants – ces substituants étant choisis dans l'ensemble constitué par les alkyles contenant de 1 à 6 atomes de carbone, les alcényles contenant de 2 à 6 atomes de carbone, les alcynyles contenant de 2 à 6 atomes de carbone, les alcoxy contenant de 1 à 6 atomes de carbone, les alkylthio contenant de 1 à 6 atomes de carbone, les alcoxycarbonyles contenant de 1 à 4 atomes de carbone dans la partie alcoxy, les alkylcarbonyles contenant de 1 à 4 atomes de carbone dans la partie alkyle, les atomes d'halogènes, le radical cyano, le radical nitro, les dialkylamino contenant de 1 à 4 atomes de carbone dans chacune des parties alkyles, les trihalogénométhyles et les alkylcarbonyloxy dont la partie alkyle contient de 1 à 4 atomes de carbone – et les trihalogénométhyles dont les halogènes sont pris dans l'ensemble comprenant Cl, Br et F.

7. Procédé selon la revendication 1 dans lequel chacun des symboles $R^1$, $R^2$ et $R^3$ est un élément de l'ensemble constitué par les alkyles en $C_1$–$C_{15}$ substitués ou non, les alcényles en $C_2$–$C_{15}$ substitués ou non, les alcynyles en $C_2$–$C_{15}$ substitués ou non, les aryles en $C_6$–$C_{20}$ substitués ou non, les hétéro-aryles, substitués ou non, qui contiennent de 2 à 20 atomes de carbone et de 1 à 4 hétéro-atomes pris dans l'ensemble constitué par N, O et S, les cycloalkyles en $C_3$–$C_{20}$ substitués ou non, les cycloalcényles en $C_3$–$C_{20}$ substitués ou non, et les hétérocycloalkyles, substitués ou non, qui contiennent de 2 à 20 atomes de carbone et de 1 à 4 hétéro-atomes pris dans l'ensemble constitué par N, O et S; $R^1$ peut en outre représenter un atome d'hydrogène; R et $R^1$ peuvent former ensemble, et avec l'atome de carbone auquel ils sont liés, un cycloalkyle en $C_4$–$C_{15}$ substitué ou non ou un hétérocycloalkyle; et les substituants dans lesdits radicaux substitués sont choisis dans l'ensemble constitué par les alkyles contenant de 1 à 6 atomes de carbone, les alcényles contenant de 2 à 6 atomes de carbone, les alcynyles contenant de 2 à 6 atomes de carbone, les alcoxy contenant de 1 à 6 atomes de carbone, les alkylthio contenant de 1 à 6 atomes de carbone, les alcoxycarbonyles contenant de 1 à 4 atomes de carbone dans la partie alcoxy, les alkylcarbonyles contenant de 1 à 4 atomes de carbone dans la partie alkyle, les atomes d'halogènes, le radical cyano, le radical nitro, les dialkylamino contenant de 1 à 4 atomes de carbone dans chacune des parties alkyles, les trihalogénométhyles et les alkylcarbonyloxy dont la partie alkyle contient de 1 à 4 atomes de carbone.